Europäisches Patentamt

European Patent Office

Office européen des brevets

(11) Publication number: **0 417 906 A1**

# EUROPEAN PATENT APPLICATION

(21) Application number: **90308824.3**

(22) Date of filing: **10.08.90**

(51) Int. Cl.5: **C12N 15/12, C12N 15/62, C07K 13/00, C12N 15/85**

The microorganisms have been deposited with the NRRL under numbers: B-15830, B-15993, B-18216, B-15929 and B-15626.

The applicant has subsequently filed a sequence listing and declared, that it includes no new matter.

(30) Priority: **11.08.89 US 392864**

(43) Date of publication of application:
**20.03.91 Bulletin 91/12**

(84) Designated Contracting States:
**AT BE CH DE DK ES FR GB GR IT LI NL SE**

(71) Applicant: **ELI LILLY AND COMPANY**
**Lilly Corporate Center**
**Indianapolis Indiana 46285(US)**

(72) Inventor: **Lai, Mei-Huei Tsai**
**2110 Pebble Beach Drive**
**Carmel, Indiana 46032(US)**
Inventor: **Belagaje, Rama M.**
**7821 Mohawk Lane**
**Indianapolis, Indiana 46260(US)**

(74) Representative: **Raynor, John et al**
**W.H. Beck, Greener & Co 7 Stone Buildings**
**Lincoln's Inn**
**London WC2A 3SZ(GB)**

(54) **Expression of a functional insect specific toxin gene in mammalian cells.**

(57) The present invention discloses a novel method for the production of a properly folded, functional insect-specific insect toxin protein in eucaryotic cells. The expression of the functional protein is achieved by the fusion of the DNA sequence encoding the structural gene product to a heterologous mammalian signal peptide. In the preferred embodiment of the invention described herein, the DNA sequence encoding the insect toxin of the scorpion Androctonus australis is joined directly to the DNA sequence encoding the human interleukin-2 signal peptide. This hybrid gene is incorporated into a eucaryotic cloning vector incorporating a selectable marker. Colonies of transformed cells identified via the selectable marker were subcultured and the culture media assayed for insecticidal activity. The resultant secreted protein demonstrated insecticidal activity against Aedes aegypti mosquito instar larvae while demonstrating no toxic effects in mice.

EP 0 417 906 A1

## EXPRESSION OF A FUNCTIONAL INSECT SPECIFIC TOXIN GENE IN MAMMALIAN CELLS

The venom of certain species of scorpion possesses certain proteins which are selectively toxic to insects. These insect toxins, although powerful neurotoxins with respect to insects, are harmless to crustaceans, arachnids, and mammals. The neurotoxic effect of the toxin causes an irreversible increase in axonal sodium permeability resulting in contractile paralysis and death of the prey. The specificity of these toxins coupled with their biodegradability make them attractive natural alternatives to chemical pesticides.

The Androctonus australis Insect Toxin (AaIT) protein is one such neurotoxin which naturally occurs in the venom of the scorpion Androctonus australis . Isolation of the AaIT protein from nature in commercially feasible quantities is impracticable if not impossible given that the protein constitutes less than 1% of total protein content of scorpion venom. This invention provides an alternative method for the production of the AaIT protein using recombinant DNA technology which results in a commercially practicable process for the production of a functional insect toxin protein.

Prior efforts to express this gene in a procaryotic environment have failed to produce a functional product. The peculiar eucaryotic system of subcellular compartmentalization and secretion are essential to the formation of a properly formed tertiary structure. The AaIT protein is highly crosslinked containing four disulfide bridges in a 70 amino acid protein. The proper formation of these bonds is critical to the functionality of the AaIT protein. Zlotkin, E. (1983) Insect Selective Toxins Derived from Scorpion Venoms, Insect Biochemistry 13 , 219-236. The AaIT protein as a secretory protein is characteristically vectorially discharged into the lumen of the endoplasmic reticulum (ER) concurrent with translation. It is apparent that the subcellular environment of the ER lumen possesses the ionic and polar character essential to proper folding of the AaIT protein. This invention demonstrates a method of producing secretory proteins in eucaryotic cell culture permitting commercially feasible production. Furthermore this system results in secretion of AaIT into the culture medium alleviating the need to lyse the expressing cells in order to isolate the AaIT protein.

Signal peptide sequences are characteristic of and essential to the proper translation and vectorial discharge of secretory proteins or those targeted for localization in subcellular membrane bound compartments. Davis, B. and Tai, P. (1980) Nature 283 , p. 433-438. A comparison of the amino acid sequences of signal peptides isolated from various sources indicate little if any conservation of primary structure. Von Heijne, G., Eur. J. Biochem. 133 , p. 12-21 (1983). Studies indicate the importance of the overall conformation of the signal peptide rather than any required primary sequence. However others in the field believe it necessary to retain the amino-terminal amino acid sequence of the protein indigenous to the signal peptide in order to maintain proper translocation. Emr, S.D., et al. , (1980) J. Cell. Biology , 86 , p. 701-711; Wiren, K. M., et al . (1988), J. Biol. Chem. , 263 , p. 19771-19777. The practice of incorporating this indigenous lead-in sequence results in a secreted product different from the true structural protein sought to be expressed. Consequentially it is necessary to later modify (if possible) the product in order to obtain the desired protein. The method of the invention described herein eliminates this step by coupling the signal sequence of one protein directly to the structural AaIT gene resulting in the production of a functional AaIT molecule without the need for subsequent modification. Additionally the absence of this lead-in sequence eliminates any possible conformational maladies occasioned by its presence.

The proper lead-in sequence is also important to the proper folding of the protein. It is believed that the conformation of the amino terminus of the protein produces a cooperative effect resulting in rapid proper folding of the protein. Thus, the use of a lead-in sequence indigenous to the signal peptide modifies the amino terminus of the protein which may affect proper folding and consequentially impairing functionality.

For the purposes of the present invention, as disclosed and claimed herein, the following terms are as defined below.

A - deoxyadenosine.

Ala - an alanine residue.

Analog - a molecule similar to another in structure and function.

Ap$^R$ - the ampicillin-resistant phenotype or gene conferring same.

Arg - an arginine residue.

Asn - an asparagine residue.·

Asp - an aspartic acid residue.

C - deoxycytosine.

Cys - a cysteine residue.

G - deoxyguanosine.

Gln - a glutamine residue.

Glu - a glutamic acid residue.

Gly - a glycine residue.

His - a histidine residue.

Ile - an isoleucine residue.

Insect Toxin - one of a class of proteinaceous toxins found in some animal venoms which have a neurotoxic effect on most insects while having no coordinate effect in arachnids, crustaceans, or mammals.

Integrating Sequence - a DNA sequence that provides for autonomous replication or chromosomal integration of a vector in a particular host cell.

Leu - a leucine residue.

Lys - a lysine residue.

Met - a methionine residue.

Nascent protein - the polypeptide produced upon translation of a MRNA transcript, prior to any posttranslational modifications.

pA - a DNA sequence encoding a polyadenylation signal.

Phe - a phenylalanine residue.

pL - a DNA segment comprising the promoter activity of the bacteriophage λ leftward promoter.

Pro - a proline residue.

Promoter - a DNA sequence that directs transcription of DNA into RNA.

rAaIT - recombinant derivative of native AaIT.

Recombinant DNA Cloning Vector - any autonomously replicating agent, including, but not limited to, plasmids and phages, comprising a DNA molecule to which one or more additional DNA segments can be or have been added.

Recombinant DNA Expression Vector - any recombinant DNA cloning vector into which a promoter has been incorporated.

Replicon - A DNA sequence that controls and allows for autonomous replication of a plasmid or other vector.

Restriction Fragment - any linear DNA sequence generated by the action of one or more restriction endonuclease enzymes.

Selectable Marker - a DNA sequence conferring drug resistance, nutritional independence, or resulting in morphological changes in the organism, that permits the selection of recombinants over the parental types.

Sensitive Host Cell - a host cell that cannot grow in the presence of a given antibiotic or other toxic compound without a DNA segment that confers resistance thereto.

Ser - a serine residue.

Signal Peptide - an amino terminal polypeptide of varying length and primary structure necessary to and characteristic of the proper vectorial discharge of proteins to be segregated in membrane bound compartments. This sequence is co- or post-translationally cleaved from the structural gene product.

Signal Recognition Particle - a cytosolic particle of undetermined composition which is believed to interact with the central region of the nascent signal peptide sequence during the initial stages of translation and halting translation until the translational apparatus interacts with membrane bound protein(s) which facilitate the vectorial discharge of the polypeptide through the membrane.

SP-IT Gene - a recombinant DNA sequence encoding a mammalian signal peptide sequence linked directly to DNA sequence encoding an insect toxin gene or a functional derivative thereof.

Structural Gene - any DNA sequence that encodes a functional polypeptide, inclusive of translational start and stop signals.

T - deoxythymidine.

$Tc^R$ - the tetracycline-resistant phenotype or gene conferring same.

Thr - a threonine residue.

Translational Apparatus - the amalgamation of those factors required for proper translation of mRNA into a polypeptide sequence, including but not limited to the ribosomal complex, mRNA, GTP, GMP, and particular regulatory factors.

Trp - a tryptophan residue.

Tyr - a tyrosine residue.

Val - a valine residue.

Figure 1: A restriction site and function map of plasmid pKC 283 produced in substantial accordance with the teaching of Example 1.

Figure 2: A restriction site and function map of plasmid pKC 283 pX produced in substantial accordance with the teaching of Example 2.

Figure 3: A restriction site and function map of plasmid pKC 283-L produced in substantial accordance with the teaching of Example 4.

Figure 4: A restriction site and function map of plasmid pKC 283-LB produced in substantial accordance with the teaching of Example 5.

Figure 5: A restriction site and function map of plasmid pKC 283 PRS produced in substantial accordance with the teaching of Example 6.

Figure 6: A restriction site and function map of plasmid pL 32 produced in substantial accordance with the teaching of Example 6.

Figure 7: A restriction site and function map of plasmid pNM 789 produced in substantial accordance with the teaching of Example 7.

Figure 8: A schematic depiction of the creation of plasmid p 120 from plasmid pNM 789 and the Pvu II-Bam HI synthetic fragment reflecting the teaching of Example 7.

Figure 9: A restriction site and function map of plasmid pL 47 produced in substantial accordance with the teaching of Example 7.

Figure 10: A restriction site and function map of plasmid pRB-IT produced in substantial accordance with the teaching of Example 9.

Figure 11: The coding sequence of the positive strand of the DNA sequence encoding the SP-IT hybrid gene. The figure also provides the corresponding amino acid sequence of the SP-IT gene product.

Figure 12: A restriction site and function map of plasmid pMSV-IT produced in substantial accordance with the teaching of Example 10.

The present invention comprises a method for producing a functional secreted insect toxin in a recombinant eucaryotic host cell, said method comprising

A. transforming or transfecting said eucaryotic host cell with a recombinant DNA expression vector comprising:

    1. an origin of replication or integrating sequence,

    2. a promoter or translational activating sequence functional in said host cell,

    3. a DNA sequence encoding a mammalian signal peptide,

    4. a DNA sequence encoding an insect toxin gene, and,

    5. a selectable marker, then

B. culturing said eucaryotic host cell under conditions suitable for the expression and secretion of said functional insect toxin.

In one embodiment of the invention primarily exemplified herein, the synthetic toxin gene comprises a DNA sequence encoding the human interleukein-2 signal peptide (IL-2-SP) in conjunction with a DNA sequence encoding the insect specific toxin of the scorpion Androctonus australis (AaIT). The present invention also comprises functional equivalents or derivatives of the polynucleotide sequence which encodes the same amino acid sequence of IL2-SP and the insect toxin. The term "functional equivalents or derivatives" is meant to include the fragments, variants, or analogs of the nucleotide sequence which encode a preprotein having the equivalent properties and biological function as the claimed preprotein.

The functional chimeric gene is composed of several factors:

a. a promoter or transcriptional activating sequence,

b. a DNA sequence encoding a mammalian signal peptide sequence located downstream of and in proper reading frame with an associated translational activating sequence inclusive of an ATG start codon, and

c. a DNA sequence encoding an insect toxin gene or derivative directly following and in proper reading frame with the signal peptide encoding sequence.

The nucleotide sequence encoding a known polypeptide sequence may be discerned using readily available genetic code sequences such as the Stanford codes for DNA ambiguities. The nucleotide sequence may be generated by reverse translation into oligonucleotide segments generated by a DNA synthesizer, such as the Applied Biosystems Model 380-A DNA synthesizer, which are then ligated according to established standard procedures. Maniatis, T. et al . (1982) Molecular Cloning - A Laboratory Manual .

The gene was constructed of two segments. It is common practice in describing the amino acid sequence of the preprotein form of a secretory protein to use the cleavage point as the numbering origin. Those amino acids upstream (i.e., the signal sequence) are termed as -1, -2, -3 etc as one moves away from the signal peptide cleavage point. These amino acids downstream are similarly termed +1, +2, etc.

The plasmid ultimately used in the production and secretion of the scorpion toxin AaIT derivative in murine cells was constructed in three steps. The first step involved the chemical synthesis and cloning of the AaIT derivative gene into plasmid pBR 322. The 226 bp synthetic fragment was created from the published amino acid sequence of the insecticidal toxin of the scorpion Androctonus australis (Zlotkin, E.

1983. Insect Biochemistry 13 , 219-236) using techniques well known in the art. The synthetic oligonucleotide fragment contained the substance of the AaIT derivative gene encoding amino acids +1 to +69 and a Bam HI endonuclease recognition sequence at both ends, the positive strand sequence of which is:

$$5'\text{-GATCCAA ATAATGAAAA AAAACGGCTA CGCTGTTGAC TCTTCT}$$
$$\text{GGCAAAGCTC CGGAATGCCT GCTGTCTAAC TACTGCAACA ACCAGTGCAC}$$
$$\text{TAAAGTTCAT TACGCTGACA AAGGCTACTG CTGCCTGCTG TCCTGCTACT}$$
$$\text{GCTTCGGCCT GAACGACGAC AAAAAAGTTC TGGAAATCTC TGACACTCGT}$$
$$\text{AAATCTTACT GCGACACTAC TATCAACTAA TAG-3'}$$

After phosphorylation of the fragment with T4 polynucleotide kinase (Pharmacia-LKB Biotechnology, Inc., 800 Centennial Avenue, Piscataway, NJ 08854) to provide 5'-phosphate groups for ligation by T4 DNA ligase, the synthetic sequence was then introduced into the Bam HI site of plasmid pBR322 (New England Biolabs, 32 Tozer Road, Beverly, MA 01915) to create plasmid pBR322-IT.

The second step involved the chemical synthesis of a DNA fragment (Segment A) encoding the 20 amino acid signal peptide of human interleukin-2 (IL2-SP) and the first 8 amino acids of AaIT with recognition sequences for endonucleases Bgl II and Hin F1 at the 5' and 3' ends, respectively. The positive strand sequence of this segment (Segment A) is:

$$\text{GAT  CTA  TAA  ATA  ATG  TAC  AGG  ATG  CAA  CTC}$$
$$\text{CTG  TCT  TGC  ATT  GCA  CTA  AGT  CTT  GCA  CTT}$$
$$\text{GTC  ACA  AAC  AGT  AAA  AAA  AAC  GGC  TAC  GCT}$$
$$\text{GTT  G}$$

Plasmid pBR322-IT was then digested with Bam HI and Hin F1 restriction endonucleases to generate segment B. DNA segment B contained the substance encoding amino acids +9 through +69 of the AaIT derivative protein, the positive strand sequence of which is:

$$\text{AC  TCT  TCT  GGC  AAA  GCT  CCG  GAA  TGC}$$
$$\text{CTG  CTG  TCT  AAC  TAC  TGC  AAC  AAC  CAG  TGC}$$

$$\text{ACT  AAA  GTT  CAT  TAC  GCT  GAC  AAA  GGC  TAC}$$
$$\text{TGC  TGC  CTG  CTG  TCT  TGC  TAC  TGC  TTC  GGC}$$
$$\text{CTG  AAC  GAC  GAC  AAA  AAA  GTT  CTG  GAA  ATC}$$
$$\text{TCT  GAC  ACT  CGT  AAA  TCT  TAC  TGC  GAC  ACT}$$
$$\text{ACT  ATC  AAC  TAA  TAG}$$

The third step in the creation of the plasmid ultimately used to express the insect neurotoxin gene involved the ligation of Segments A and B which created a hybrid gene encoding the signal peptide of human interleukin-2 and the scorpion insecticidal neurotoxin AaIT derivative with recognition sequences for endonucleases Bgl II and Bam HI at the respective 5' and 3' ends. The hybrid gene was then inserted into plasmid pL47 at the Bgl II and Bam HI sites.

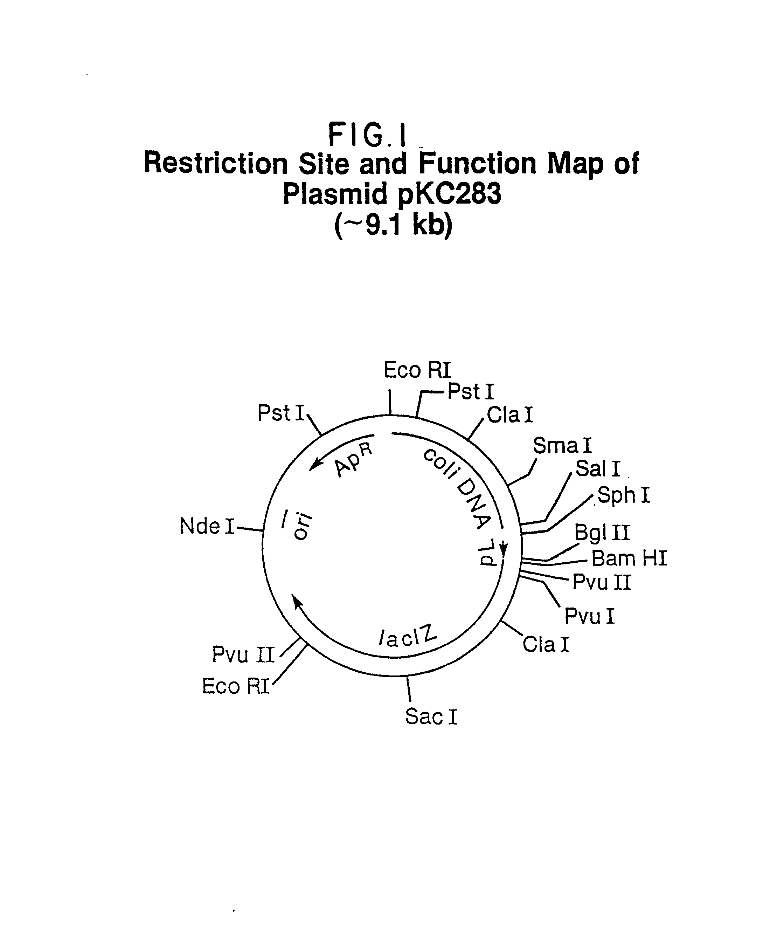

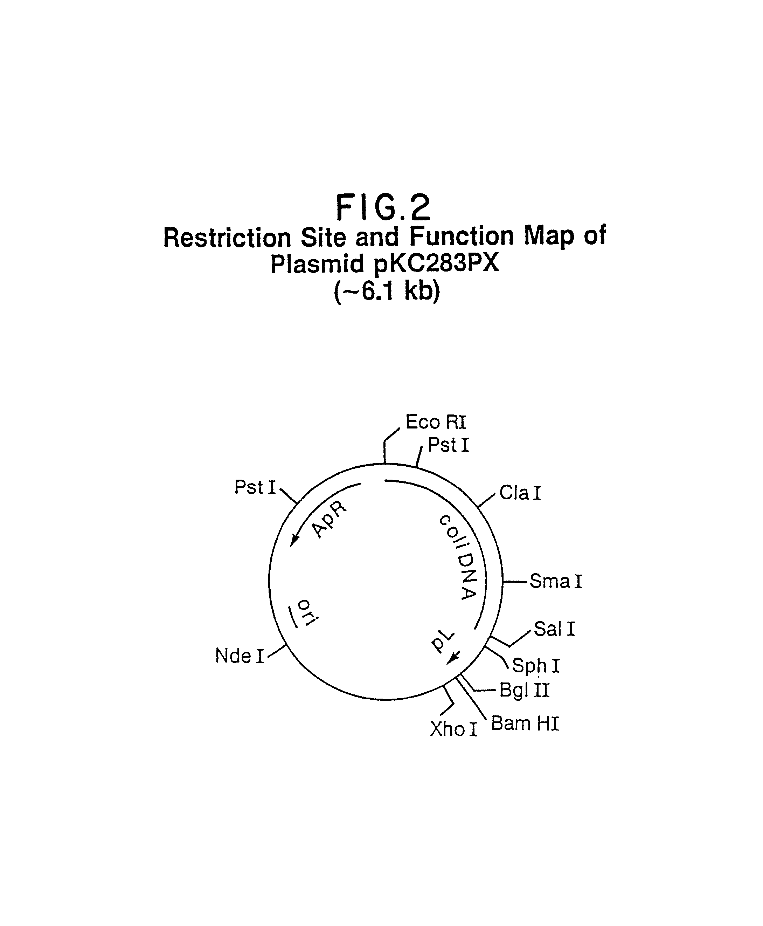

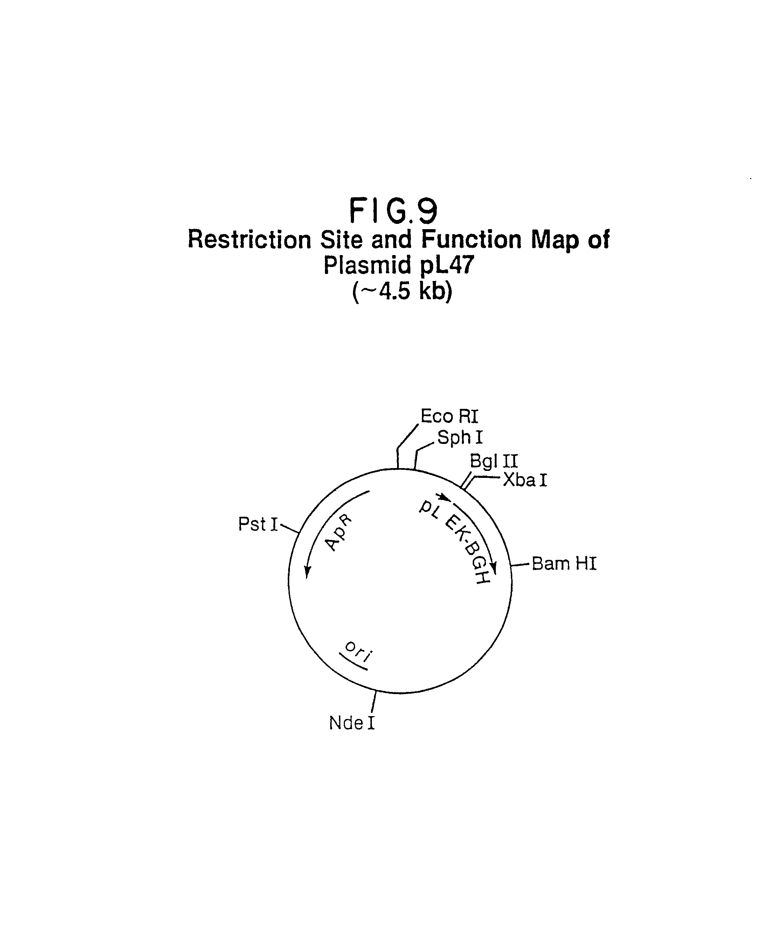

A restriction site and function map of plasmid pL47 is found in Figure 9 of the accompaning drawings. Plasmid pL47 was constructed by first isolating plasmid pKC283 from E. coli K12 BE1201/pKC283. This culture may be obtained from the NRRL under accession number NRRL B-15830. Plasmid pKC283 comprises a hybrid lpp -pL promoter of bacteriophage λ. This plasmid is obtained from E. coli K12 BE1201 cells because these cells comprise a temperature sensitive cl repressor integrated into the cellular DNA. The unneeded lac Z portion of plasmid pKC283 was excised by digesting the plasmid with restriction enzyme Pvu II. Specific DNA linkers were then added to the digested DNA to convert the Pvu II sites into a single Xho I site, which created plasmid pKC283PX. [Detailed descriptions of the isolation of plasmids pKC283 and pKC283PX are presented respectively in Examples 1 and 2.] Restriction site and function maps of plasmids pKC283 and pKC283PX are presented in Figures 1 and 2 of the accompanying drawings. Plasmid pKC283PX is cloned using standard techniques into E. coli K12 MO(λ⁺). E. coli K12 MO(λ⁺) is available from the NRRL under the accession number NRRL B-15993.

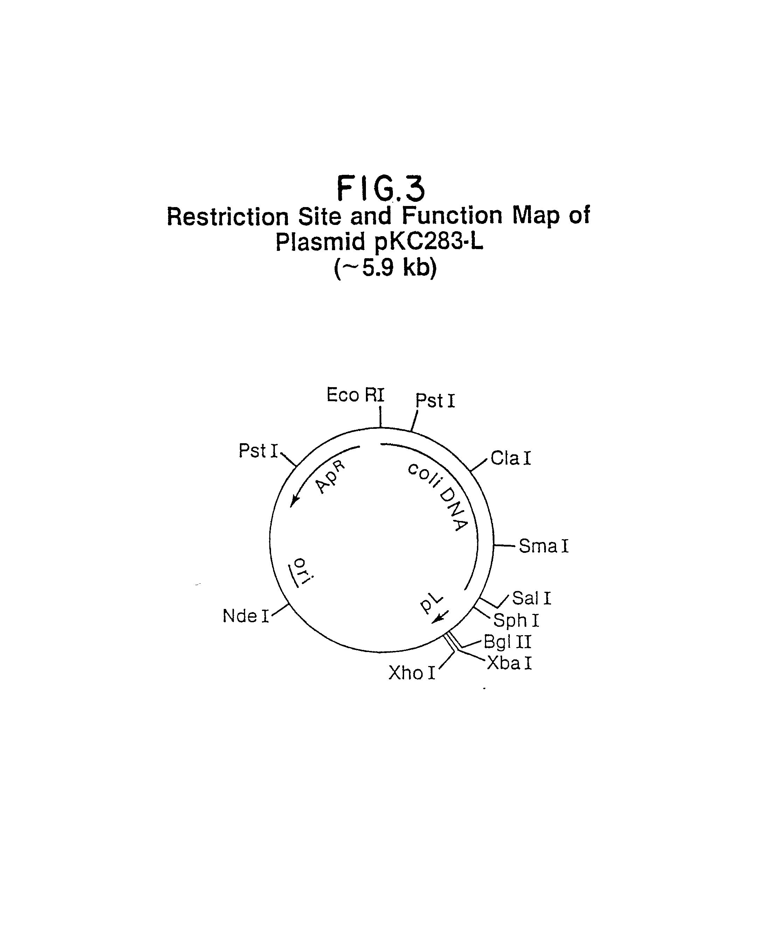

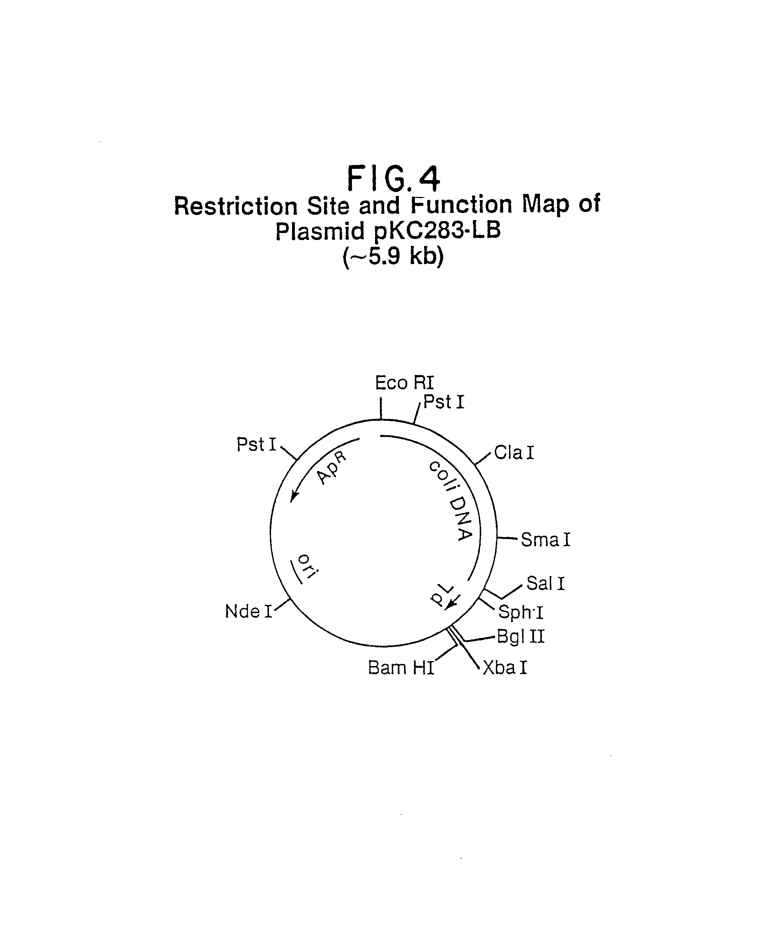

Plasmid pKC283PX was next digested with restriction enzymes Bgl II and Xho I. After the vector was purified, DNA linkers with Bg III and Xho I ends were ligated into the vector to form plasmid pKC283-L. The Bgl II-XhoI linker also contained an XbaI site. The Xho I site of plasmid pKC283-L was next converted into a Bam HI site. This was accomplished by a total digestion of plasmid pKC283-L with restriction enzyme Xho I, followed by treatment with Klenow, then addition of Bam HI linkers, to form plasmid pKC283-LB. Detailed descriptions of the construction of plasmids pKC283-L and pKC283-LB are presented respectively in Examples 4 and 5. Restriction site and function maps of plasmids pKC283-L and pKC283-LB are presented in Figures 3 and 4 of the accompanying drawings.

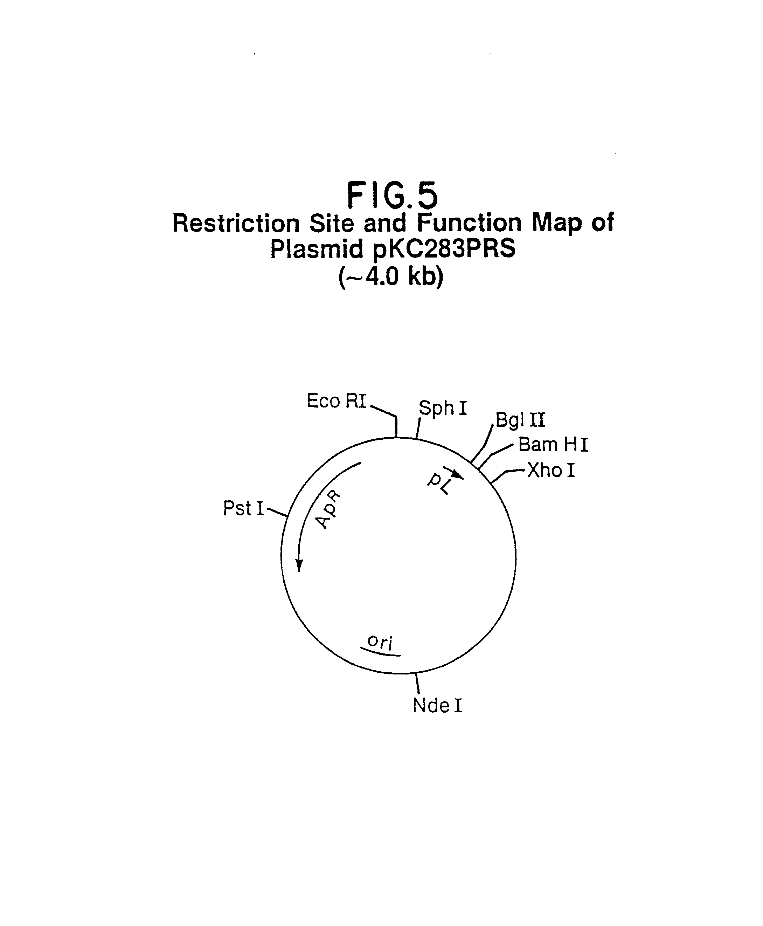

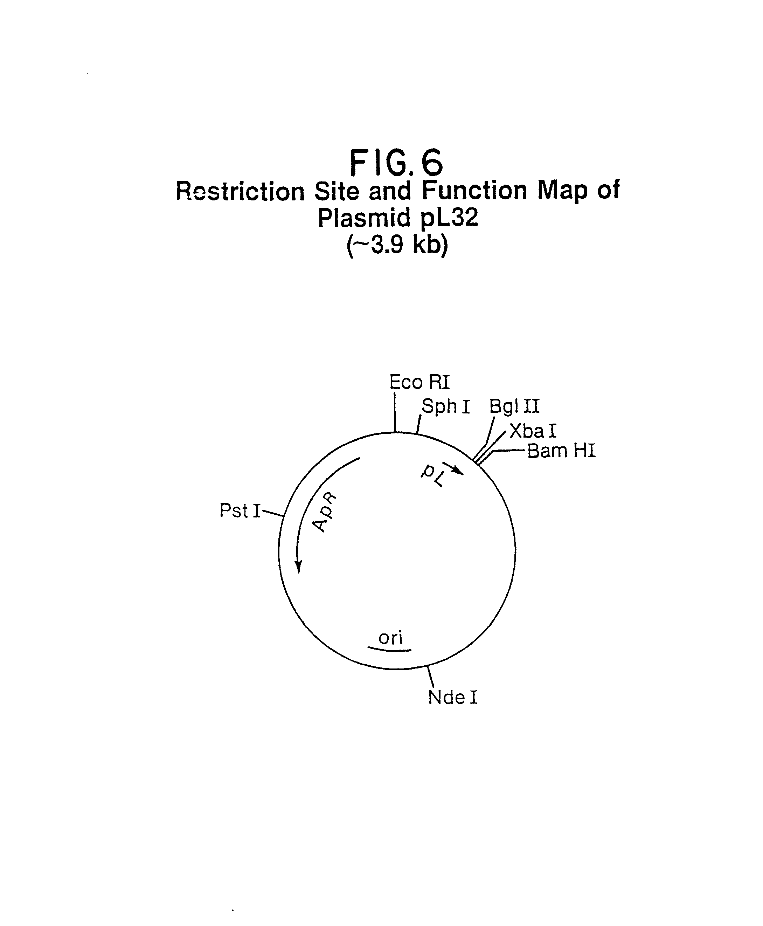

The extraneous E. coli DNA was next excised from plasmid pKC283PX by total digestion with restriction enzyme Sal I, followed by treatment of the ~4.0 kb vector with Klenow, then addition of Eco RI linkers. Upon recircularization via ligation, this formed plasmid pKC283PRS. Plasmid pKC283PRS was then digested with restriction enzymes Pst I and Sph I and the ~0.85 kb Pst I-Sph I restriction fragment was isolated. In an analogous manner, plasmid pKC283-LB was digested with restriction enzymes Pst I and Sph I and the ~3.0 kb fragment was isolated. The ~0.85 kb Pst I-Sph I fragment of pKC283PRS was then ligated into the ~3.0 kb Pst I-Sph I vector fragment of pKC283-LB to form plasmid pL32. Detailed descriptions of the construction of plasmids pKC283PRS and pL32 are presented in Example 6. Restriction site and function maps of plasmids pKC283PRS and pL32 are presented in Figures 5 and 6 of the accompanying drawings.

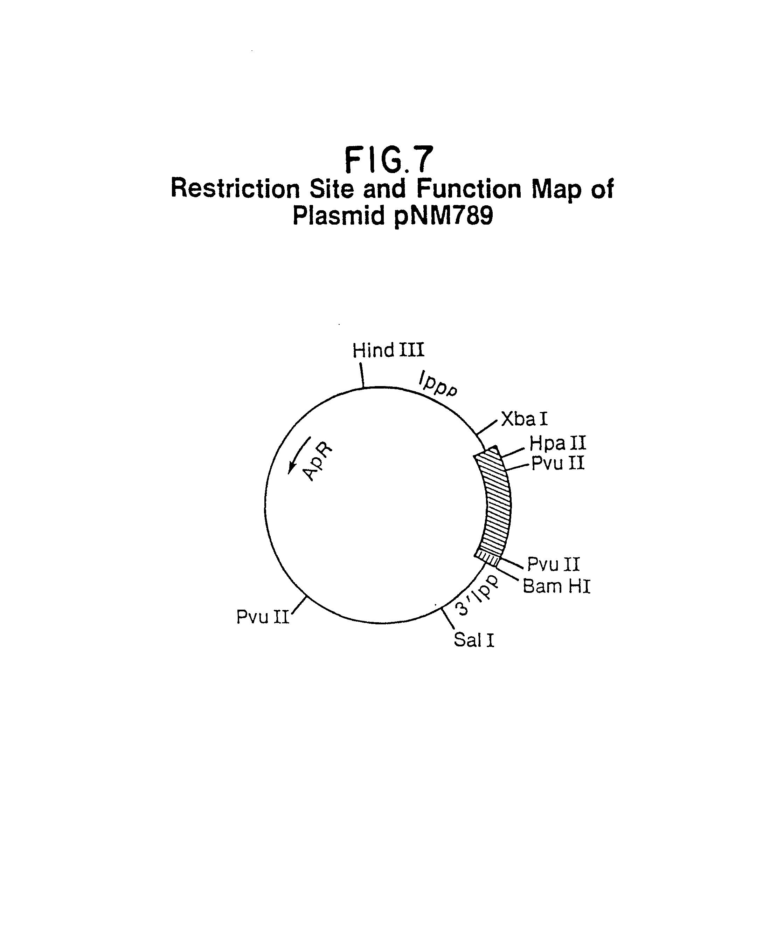

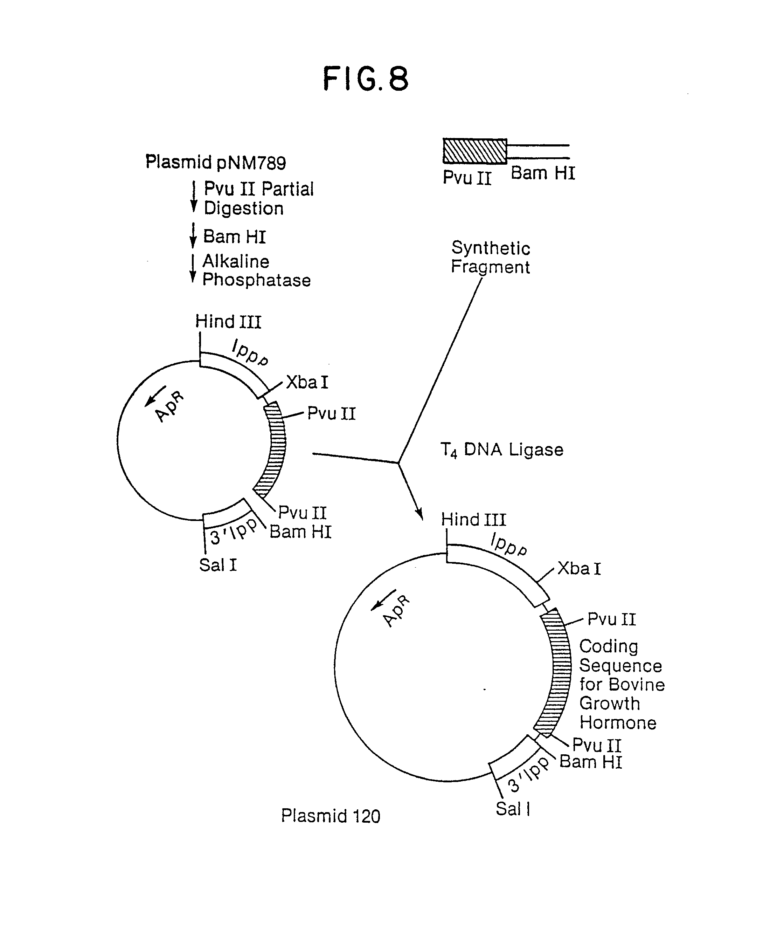

Next, plasmid pNM789 is obtained from the NRRL in E. coli K12 RV308/pNM789 under the accession number B-18216. A restriction site and function map of plasmid pNM789 is presented in Figure 7 of the accompanying drawings. Plasmid pNM789 was partially digested with restriction enzyme Pvu II, fully digested with restriction enzyme Bam HI, then treated with alkaline phosphatase. Next, a new Pvu II-BaM HI linker was ligated into the digested, phosphatased vector pNM789 to form plasmid 120. Plasmid 120 was then totally digested with restriction enzymes Xba I and Bam HI and the ~0.6 kb Xba I-Bam HI EK-BGH-encoding restriction fragment was isolated. Plasmid pL32 was also digested with restriction enzymes Xba I and Bam HI and the ~3.9 kb vector fragment was isolated. The ~0.6 kb Xba I-Bam HI fragment of plasmid 120 was then ligated into the ~3.9 kb vector fragment of plasmid pL32 to form plasmid pL47. Detailed descriptions of the construction of plasmids 120 and pL47 are presented in Examples 6 and 7. Restriction site and function maps of plasmids 120 and pL47 are presented respectively in Figures 8 and 9 of the accompanying drawings.

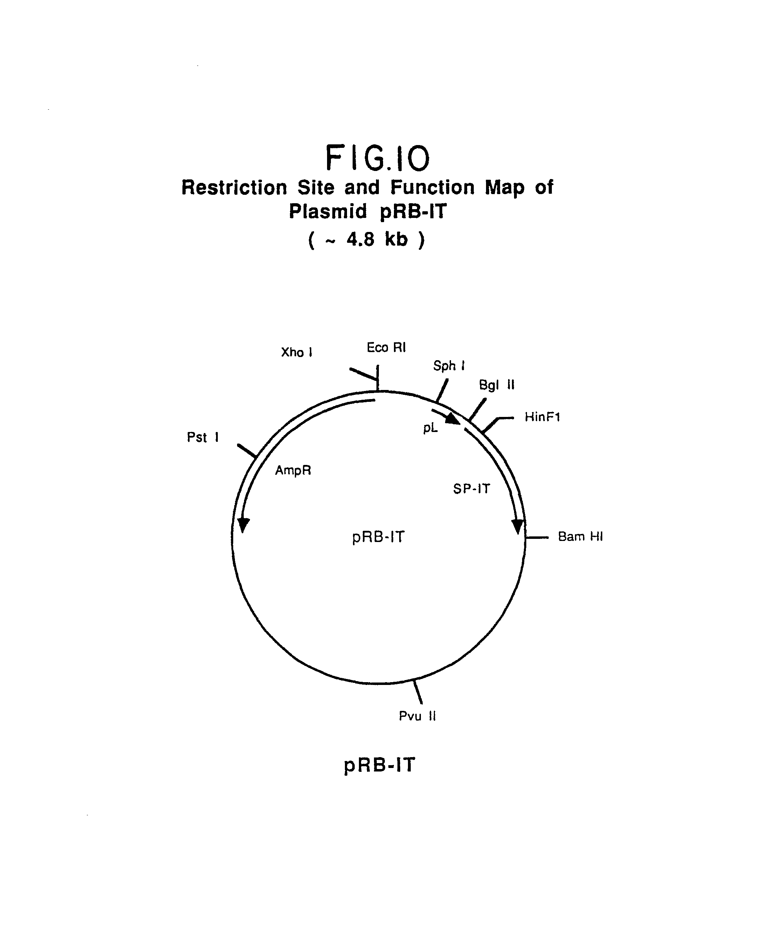

The segments A and B, when ligated together, generated a chimeric gene that coded for a hybrid polypeptide of IL2-sp-AaIT derivative. In the preferred embodiment exemplified herein, the chimeric gene encoding the IL2-SP-AaIT derivative propeptide with a Bgl II recognition sequence at the 5′ end and a Bam HI recognition sequence at the 3′ end was cloned into the procaryotic cloning vector pL47 utilizing the unique Bam HI site of pL47 to yield plasmid pRB-IT. A restriction map of pRB-IT is shown in Figure 10. The plasmid was amplified by transformation into E. coli utilizing standard proceudres. The plasmid was isolated by cesium chloride density gradient centrifugation.

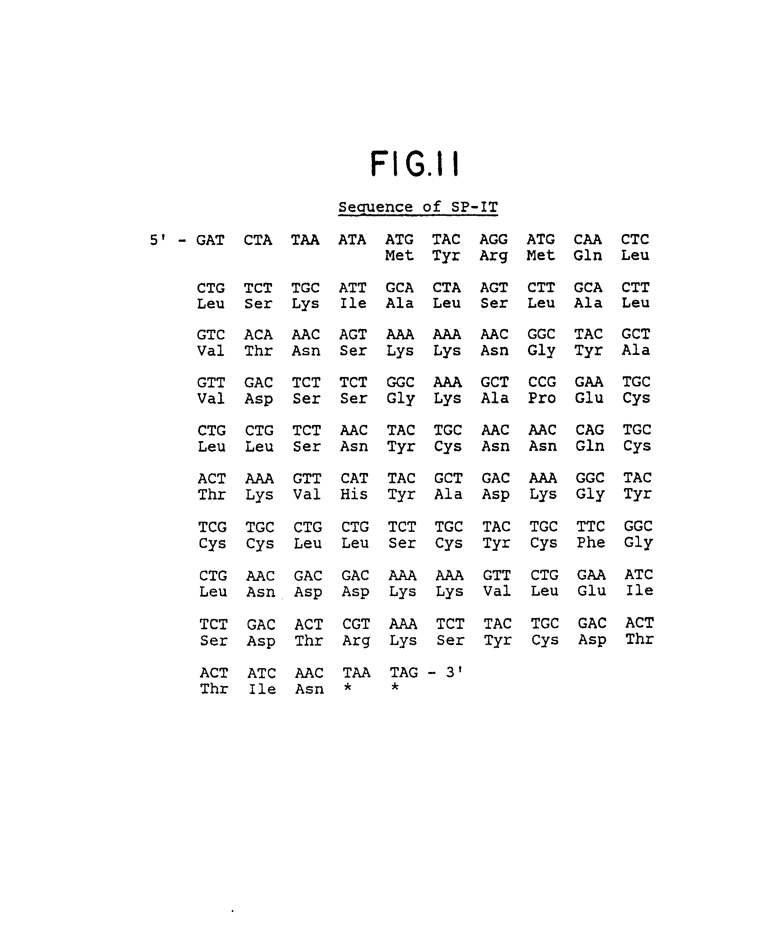

The pRB-IT plasmid was cut with Bgl II and Bam HI to isolate the 285 bp fragment encoding the SP-IT gene, the sequence of which is shown in Figure 11. The 285 bp fragment was isolated by agarose gel electrophoresis. The DNA sequence encoding the SP-IT gene was next inserted into an expression vector, such as a plasmid or viral vector capable of transforming eucaryotic cells. A preferred vector was the retroviral vector which comprises a plasmid having all or a portion of a proviral sequence incorporated into its genome. The retroviral genes are flanked by two long terminal repeat sequences. The 5′ and 3′ long terminal repeat sequences promote transcription and polyadenylation of viral mRNA's. The synthetic SP-IT gene was inserted in the proviral portion of the plasmid under transcriptional control of the viral long terminal repeat (LTR) sequence. In one embodiment of the present invention the SP-IT gene was inserted into the preferred retroviral vector pMSV. The pMSV (disclosed in U.S. Patent No. 4,775,624, issued October 4, 1988, the entire teaching of which is herein incorporated by reference) plasmid contains the entire proviral sequence of Mo.MSV, a murine retrovirus (Stratowa, C., et al . (1982) EMBO J. , 12; 1973-78).

A recombinant retroviral vector is comprised of four elements:

A. a 5′ LTR from the retrovirus followed by DNA containing the retroviral packaging signal sequence;

B. the particular gene sought to be expressed, i.e. the insect toxin gene, SP-IT;

C. a selectable marker gene; and

D. a 3′ LTR which contains an intact LTR or a deletion in the viral enhancer region, or deletions in both the viral enhancer and promoter regions. A recombinant retroviral vector is capable of transferring genes into a carrier cell line wherein transformants are identified on the basis of the selectable marker. Individual colonies may be picked and subcultured to obtain a single transformed cell line.

Several types of marker genes are available which confer morphological variations or chemical dependence or independence. A preferred marker is the v-mos oncogene, which is a transformation specific gene of the Moloney murine sarcoma virus (Mo-MSV). Van Beveren et al . Nature , 289 , 258-262 (1981). The v-mos oncogene transforms the host cell inducing readily visible morphological changes. For example, untransformed 3T3 cells are flat and subject to contact inhibition. However v-mos transformed 3T3 cells are rounded or spindle shaped, highly refractile and are not contact inhibited. Such v-mos /3T3 transformants appear several days after transfection and by two weeks the foci of the transformants are clearly visible. By placing the SP-IT gene under the same LTR transcriptional control as the v-mos gene, recombinants expressing the SP-IT gene may be isolated on the basis of the morphological changes characteristic of v-mos .

Other selectable markers are available. Genes which confer antibiotic resistance are routinely used to isolate transformants by the transformed cell's resistance to the antibiotic present in the culture medium. For example neomycin or hygromycin resistance genes linked to the gene sought to be inserted and expressed allow isolation of transformants by culture of the cells in a media containing otherwise toxic levels of antibiotic.

Insertion of the hybrid gene into the retroviral plasmid was accomplished by restriction endonuclease digestion and ligation. The linearized chimeric gene sequence, isolated by Bgl II and Bam HI digestion of the pRB-IT plasmid, was incorporated into the pMSV plasmid utilizing the unique Bg III site of pMSV. The resulting plasmid, pMSV-IT was amplified in E. coli using procedures in substantial accord with Example 3. The pMSV-IT plasmid was isolated by CsCl density gradient centrifugation.

The retroviral vector was employed to introduce the SP-IT gene into the host cell. The plasmid containing the SP-IT gene, the selectable marker, and 5′ and 3′ LTR sequences was transmitted into the host cell by transfection methods such as the calcium-phosphate procedure M. Wigler, et al. Cell , 14 , 725 (1978), M. T. Lai and I. M. Verma, Virology , 104 407-417 (1980). Transcription and translation of these plasmid genes was accomplished by the transcriptional and translational machinery of the host cell. The transfected cells identified by the selectable marker are subcultured to produce large quantities of the transformed cells.

To isolate the insect toxin and to insure its proper extracellular secretion, the cells were cultured to 80-90% confluency. The serum-containing medium was then replaced with serum-free DMEM (Dulbecco's modified Eagle's medium). The cells were then incubated for 24 hours. The tissue culture fluids were then collected, cleared of cell debris by low speed centrifugation and concentrated using an Amicon ultrafiltration apparatus fitted with a YM 10 membrane filter. Aliquots for later testing were collected when the concentration was approximately 5-fold, 20-fold, 50-fold, 100-fold, 200-fold and 300-fold.

The functionality of the secreted insect toxin was measured by the ability of the concentrated culture media of pMSV-IT transformed NIH/3T3 cells (ATCC CCL92) to kill yellow fever mosquito ( Aedes aegypti ) instar larvae. Yellow fever instar larvae were obtained in substantial accordance with the teaching of Example 12. Approximately 10-15 larvae in 50 μl of hatching broth were exposed to an equal volume of concentrated culture broth obtained from pMSV-IT transformed 3T3 cells (DC-1). The mortailty was monitored at 2, 4, and 24 hours after exposure to the concentrated culture media. Table 1 illustrates the results of this procedure. The data clearly demonstrated the ability of the culture media of pMSV-IT transformed cells (DC-1) to kill mosquitos in a concentration dependent fashion. The control samples of pMSV-transformed 3T3 cells (DB-2) did not show such insecticidal activity, demonstrating that insecticidal activity was a direct result of transformation of 3T3 cells by SP-IT gene. The results indicated that the AaIT derivative protein was properly folded and secreted, resulting in the production of a functional secretory AaIT derivative protein without further chemical or artificial modification.

## Table 1

### Insecticidal Activity of rAaIT
### Tested in Mosquito Larvae

| Source of culture fluid | Mortality of mosquito larvae in broth containing equal volume of tissue-culture fluid concentrated | | | | | | |
|---|---|---|---|---|---|---|---|
| | 1X | 5X | 20X | 50X | 100X | 200X | 300X |
| DC-1 cells[a] | - | - | -/+[c] | +[c] | ++[c] | +++[c] | +++[c] |
| DB-2 cells[b] | - | - | - | - | - | - | - |

[a]NIH/3T3 cells transformed by pMSV-IT

[b]NIH/3T3 cells transformed by pMSV

[c]Mortality of mosquito larvae was recorded 2, 4, and 24 hours after introduction of toxin to insects
-/+: less than 100% kill in 24 hours
+: 100% kill in 24 hours
++: 100% kill in 4 hours
+++: 100% kill in 2 hours

[d]Observed one week later

Signal peptides are characteristic of those proteins which are secreted or localized into particular subcellular compartments. The signal peptide facilies the orderly subcellular localization or secretion by providing 3 primary functions:

1. arresting protein synthesis via interaction with the Signal Recognition Particle (SRP),

2. facilitating vectorial discharge through membranes, and

3. providing a point for proper protease activity at the -1 - +1, signal sequence-structural gene, interface.

Although all known signal peptides function similarly, signal peptides obtained from various sources demonstrate little if any conservation of amino acid sequence. von Heijne, G., (1983) Eur. J. Biochem 133, 17-21. However, when viewed in light of secondary and tertiary structural elements and hydrophobic/hydrophilic interactions, there are regions of homology in the signal peptide coincident with the three aforementioned functions. The three conserved regions of structural homology are termed the (a) amino terminal region, (b) the central region and (c) the carboxyl terminal region.

The amino terminal region is characterized by a net positive charge. A net negative charge will inhibit processing. This region is not sensitive to changes in length of less than six amino acids. However addition or subtraction of 10 to 13 amino acids will prevent translocation. Although length does not appear critical, the physical distance of the amino terminal region with respect to other regions of functional homology is important to signal peptide function. Additionally, while there is no particular characteristic amino acid sequence, the amino acid residues present in this region often indicate the presence of a beta-turn. The amino terminal region of the signal peptide is generally believed to be associated with the proper translocation of the polypeptide through the membranes.

The central region of the signal peptide is characterized by a high concentration of those residues favoring the formation of the $\alpha$-helix conformation. This area is strongly hydrophobic. Again, there is no conserved amino acid sequence in this region. This area is generally believed to interact with the signal recognition particle (SRP) which arrests translation pending interation with membrane bound proteins which facilitate the vectorial discharge across the membrane.

The carboxyl terminal region is characterized by a high degree of conservation at positions -1 and -3. Position -1 must be a small residue usually alanine, serine, glycine, cysteine, threonine or glutamine. The -3 position residue must not be aromatic, charged, or large and polar. Additionally proline is excluded from the -3 to -1 region. Von Heijne, G. (1986) Nucleic Acids Research , 14 , 4683-90.

Taken together these elements may demonstrate that proper signal sequence function is due primarily to the characteristic secondary and tertiary structure rather than any particular characteristic primary structure. However, others persist in the belief that features essential to the proper cleavage and function of the signal peptide are associated with primary structure of the signal peptide and the first several amino acid residues of the protein normally associated with the particular signal peptide. See Emr, S.D., et al. (1980) J. Cell Biol . 86 701-711 and Wiren, K.M., et al. , (1988) J. Biol. Chem. , 263 , 19771-19777. However, these examples neglect the fundamental interplay of primary, secondary, and tertiary structure. In employing a lead-in sequence indigenous to the signal peptide composed of the first few amino acids of the polypeptide normally associated with the signal sequence, the translation product is not truly the protein sought but a derivative of the protein with an amino terminal "lead-in" sequence. Such alterations of the amino terminus although sometimes harmless, may, depending on the peculiarities of the protein desired, lead to an inactive structural gene product.

The method described in this invention demonstrates a path which results in the structural gene product being expressed without an amino terminal lead-in sequence of human interleukin-2. This method prevents potential folding distortions created by the lead-in sequence. This method also negates the need for any further chemical processing to remove this sequence.

The possible effects on proper folding are particularly important with respect to the insect toxin proteins. The AaIT protein contains four disulfide cross bridges. The proper formation of these disulfide bonds is critical to the functionality of the protein. Zlotkin, E. (1983) Insect Biochem. , 13, 219-236. The importance of the eucaryotic signal sequence expression system associated with secretory proteins is demonstrated by the failure of efforts to produce a functional insect toxin protein in E. coli. The insect toxin proteins are secretory proteins and as such are normally discharged cotranslationally into the lumen of the endoplasmic reticulum (ER). It is apparent that there are electrical, hydrophilic and/or structural consequences of cotranslational discharge into the ER which are necessary to insure proper folding of the insect toxin protein and preserve functionality. This invention provides a method of producing functional secretory proteins in a recombinant DNA system preserving those conditions necessary to proper expression of a functional gene product apart from the signal peptide indigenous to the structural gene product.

As demonstrated by the foregoing discussion, replacement of the human interleuken-2 signal peptide sequence with other mammalian secretory signal peptide sequences should result in proper tertiary structure formation and extracellular translocation of the AaIT gene product. The amino acid sequences of a variety of such signal peptides are illustrated in von Heijne's article "Patterns of Amino Acids near Signal-Sequence Cleavage Sites", Eur. J. Biochem. 133, 17-21 (1983) coupled with the subsequent article by the same author, "A new method for predicting signal sequence cleavage sites", Nucleic Acids Research 14 , 4683-4690 (1986), the entire teaching of which are herein incorporated by reference, demonstrate the functional similarity of mammalian signal peptide sequences. Therefore, the AaIT coding sequence could be operably linked to other signal peptide coding sequences derived from secretory proteins resulting in an active secreted AaIT insect toxin or derivative thereof. Examples of such functional combinations would include the DNA sequence encoding a functional insect toxin gene operably linked to the DNA sequence encoding the rat insulin II signal peptide, or the DNA sequence encoding the rat growth hormone signal peptide, or the DNA sequence encoding the human growth hormone signal peptide.

Although the proper extracellular localization of secretory proteins is directed principally by the signal peptide sequence, the functionality of the protein gene product is ultimately derived from its primary amino acid sequence. Certain amino acid residues are characteristic of secondary structural elements such as the α-helix and β-pleated sheet. Similarly, the physical properties of certain amino acids will affect tertiary structure. The formation and stabilization of a proper tertiary protein structure is primarily due to 4 types of interactions: (1) hydrogen bonds between peptide groups as in α-helix or β-pleated sheets, (2) hydrogen bonds between amino acid side chains, (3) hydrophobic interactions between non-polar amino acid side chains, and (4) ionic bonds between positively and negatively charged side chains. Lehninger, A., Biochemistry , 2d.Ed., p. 142 Worth Publishing, N.Y. (1975). However, more than one amino acid may have similar physical properties with respect to the aforementioned factors. Therefore, substitution of an amino acid residue(s) possessing similar physical properties to the amino acid indigenous to the native protein would generally produce a functionally congruent protein. While it is true that the presence of a certain amino acid is essential to protein function as is the case with amino acids involved in enzymatic active sites, the vast majority of the primary sequence is not required to be amino acid specific in order to preserve functionality. Similarly elimination or addition of one or more amino acids may yet result in a functional protein.

The AaIT protein is no exception. Minor alterations of amino acids in non-structurally significant regions of the AaIT protein would produce functionally similar if not identical insect toxins. Such AaIT derivatives would be equally well produced by the expression system in the instant application. The production of such functional derivatives is demonstrated in the instant application. A comparison of the precise amino acid sequence expressed herein with the native AaIT protein indicates that the protein expressed herein lacks an isoleucine residue at the carboxyl terminus. However, this AaIT derivative retains the functionality properties of the native AaIT toxin.

Posttranslational modification of mammalian proteins varies among mammalian cell lines presumably due to the presence of certain proteases. Ramabhadran, T.V., et al ., Host Cell-specific Variations in the Posttranslational Modification of Engineered Proteins, Gene Transfer Vectors for Mammalian Cells, p. 85-93, Miller, J., Calos, M. eds., Cold Spring Harbor, 1987.

Although many mammalian proteins undergo extensive posttranslational modification through glycosylation, etc. which may be essential to function, the AaIT protein undergoes only a single posttranslational processing step, i.e., cleavage of the signal peptide from the preprotein form. As previously demonstrated, the signal sequences of mammalian signal peptides, particularly with respect to the conformation of the critical cleavage zone, are nearly universal with respect to overall structure and function, von Heijne, G. supra . Furthermore, there is no particular homology of signal sequence cleavage sites in various secretory proteins of a particular organism. It is apparent that the function of the cleavage of signal peptides from preproteins is a nearly universal function and not host cell specific. Therefore, proper cleavage of the signal sequence from pre-AaIT is able to be achieved in nearly any mammalian cell line.

Others have demonstrated proper signal sequence cleavage of non-native secretory proteins in various cell lines. Tanaquchi, T., et al . for example reports proper cleavage of the signal peptide from human interleukin-2 in cultured monkey cells. Nature , 304 , 305-310 (1983). Therefore the preprotein form of AaIT will be properly modified posttranslationally in other popular mammaliam cell lines such as HeLa, Av12, 3T3, BHK and 293.

In addition to AaIT, crude venom of scorpion A. australis also contains three mouse toxins (AaMT). One of them, $AaMT_1$ and AaIT share identical localizations of three of the four disulfide bridges. Zlotkin, E. (1983) Insect Biochem. 13 , 219-236. It was proposed that the difference in the localization of the fourth disulfide bridge in AaIT molecule may be critical to the neurotoxic selectivity of AaIT. Id . Therefore, it was important to demonstrate that AaIT produced by pMSV-IT transformed 3T3 cells (DC-1) was selectively toxic to insects and harmless to mammals.

The toxicity study in mice was carried out with the highest (300x) concentrates of culture fluids from both pMSV-IT-3T3 cells and pMSV transformed 3T3 (DB-2) control cells. The amount of AaIT in the 300x pMSV-IT-3T3 (DC-1) concentrate was estimated to be approximately 15 $\mu$g/ml. Approximately 0.5 ml of each concentrate was administered intravenously to each of the 3 ICR (Charles River Laboratories, Inc. 251 Ballardvale Street, Wilmington, MA 01887) female mice per group. The dose of AaIT derivative given to the test animals was estimated to be around 0.3-0.4 $\mu$g/gram body weight. Due to unavailability of purified scorpion mouse toxins, a commercial preparation (Sigma, P.0. Box 14508, St. Louis, MO 63178) of crude venom of A. australis was used as positive control. A dosage of 15-20 $\mu$g of crude venom was administered to each mouse. The dose was roughly equivalent to 2 x $LD_{50}$ reported in literature. Zlotkin et al ., (1971) Biochimie 53 , 1073-1078. Mortality and signs of toxicity (such as hypoactivity, lethargy, comatose, hind leg paralysis, tremor, diarrhea and other symptoms) were observed hourly on the day of dosing and daily for two weeks. The results are summarized in Table 2. At the end of the observation period, mice that had been injected with either 300x pMSV-IT-3T3 (DC-1) concentrate or pMSV-3T3 (DB-2) concentrate remained healthy and showed no signs of toxicity. The animals that had received scorpion venom generally died within a few hours after injection. Although no purified mouse toxins were available for a parallel experiment in our study, a rough comparison may be made with the results reported by Zlotkin et al . Id . They demonstrated that $LD_{50}$ of purified mouse toxins ($AaMT_1$, $AaMT_2$ and $AaMT_3$) of scorpion A. australis ranged from 0.18 ug to 0.45 $\mu$g per mouse. If AaIT produced by pMSV-IT (DC-1) cells was toxic to mice, the dose (7-8 $\mu$g per mouse) administered in our study should result in some clinical symptoms of toxicity, if not lethality. Yet, no signs of toxicity were ever observed in both groups of mice in a two-week observation period. Therefore, we conclude that AaIT synthesized by pMSV-IT-3T3 (DC-1) cells behaved like native AaIT regarding these two important activities of the toxin.

Table 2

| Toxicity of rAaIT Tested in Mice[a] | | |
|---|---|---|
| Compound | Mortality[b] | Toxicity[b] |
| (A) DC-1 culture medium[c] (300X concentration) | 0/3 | 0/3 |
| (B) DB-2 culture medium (300X concentration) | 0/3 | 0/3 |
| (C) Crude Venom[d] | 3/3 | 3/3 |

[a]Three ICR female mice per compound.
[b]Observed for two weeks for groups (A) and (B), one day for group (C)
[c]Estimated rAaIT used ~ 0.3-0.4 μg.g body weight
[d]Purchased from Sigma. Dosage used ~ 1 μg.g body weight

The following examples further illustrate and describe the invention disclosed herein. The invention is not limited in scope by reason of any of the following Examples; sources of reagents or equipment are provided merely for convenience and in no way limit the invention. Both an explanation of and the actual procedures for constructing the invention are described where appropriate.

Example 1

Isolation of Plasmid pKC283

Lyophils of E. coli K12 BE1201/pKC283 are obtained from the Northern Regional Research Laboratory, Peoria, Illinois 61604, under the accession number NRRL B-15830. The lyophils are decanted into tubes containing 10 ml LB medium (10 g Bacto-tryptone, 5 g Bacto-yeast extract, and 10 g NaCl per liter; pH is adjusted to 7.5) and incubated two hours at 32°C, at which time the cultures are made 50 μg/ml in ampicillin and then incubated at 32°C overnight. The E. coli K12 BE1201/pKC283 cells were cultured at 32°C, because the cells comprise a temperature-sensitive cI repressor gene integrated into the cellular DNA. When cells that comprise a wild-type lambda pL repressor gene or do not comprise a lambda pL promoter are utilized in this plasmid isolation procedure, as described in subsequent Examples herein, the temperature of incubation is 37°C.

A small portion of the overnight culture is placed on LB-agar (LB medium with 15 g/l Bacto-agar) plates containing 50 μg/ml ampicillin in a manner so as to obtain a single colony isolate of E. coli K12 BE1201/pKC283. The single colony obtained was inoculated into 10 ml of LB medium containing 50 μg/ml ampicillin and incubated overnight at 32°C with vigorous shaking. The 10 ml overnight culture was inoculated into 500 ml LB medium containing 50 μg/ml ampicillin and incubated at 32°C with vigorous shaking until the culture reached stationary phase.

The following procedure is adapted from Maniatis et al., 1982, Molecular Cloning (Cold Spring Harbor Laboratory).

The cells were harvested by centrifugation at 4000 g for 10 minutes at 4°C, and the supernatant was discarded. The cell pellet was washed in 100 ml of ice-cold STE buffer (0.1 M NaCl; 10 mM Tris-HCl, pH 7.8; and 1 mM EDTA). After washing, the cell pellet was resuspended in 10 ml of Solution 1 (50 mM glucose; 25 mM Tris-HCl, pH 8.0; and 10 mM EDTA) containing 5 mg/ml lysozyme and left at room temperature for 10 minutes. Twenty ml of Solution 2 (0.2 N NaOH and 1% SDS) were then added to the lysozyme-treated cells, and the solution was gently mixed by inversion. The mixture was incubated on ice for 10 minutes.

Fifteen ml of ice-cold ·5 M potassium acetate, pH 4.8, were added to the lysed-cell mixture and the solution mixed by inversion. The solution was incubated on ice for 10 minutes. The 5 M potassium acetate solution was prepared by adding 11.5 ml of glacial acetic acid to 28.5 ml of water and 60 ml of 5 M potassium acetate; the resulting solution is 3 M with respect to potassium and 5 M with respect to acetate.

The lysed cell mixture was centrifuged in a Beckman SW27 (or its equivalent) at 20,000 rpm for 20 minutes at 4°C. The cell DNA and debris formed a pellet on the bottom of the tube. About 36 ml of supernatant were recovered, and 0.6 volumes of isopropanol were added, mixed, and the resulting solution left at room temperature for 15 minutes. The plasmid DNA was collected by centrifugation at 12,000 g for 30 minutes at room temperature. The supernatant was discarded, and the DNA pellet was washed with 70% ethanol at room temperature. The ethanol wash was decanted, and the pellet was dried in a vacuum desiccator. The pellet was then resuspended in 8 ml of TE buffer (10 mM Tris-HC1, pH 8.0, and 1 mM EDTA).

Eight grams of CsCl were added to the DNA solution. About 0.8 ml of a 10 mg/ml solution of ethidium bromide in water were added for each 10 ml of CsCl-DNA solution. The final density of the solution was about 1.55 g/ml, and the ethidium bromide concentraton was about 600 $\mu$g/ml. The solution was transferred to a Beckman Type 50 centrifuge tube, filled to the top with paraffin oil, sealed, and centrifuged at 45,000 rpm for 24 hours at 20°C. After centrifugation, two bands of DNA were visible in ordinary light. After removing the cap from the tube, the lower DNA band was removed by using a syringe with a #21 hypodermic needle inserted through the side of the centrifuge tube.

The ethidium bromide was removed by several extractions with water-saturated 1-butanol. The CsCl was removed by dialysis against TE buffer. After extractions with buffered phenol and then chloroform, the DNA was precipitated, washed with 70% ethanol, and dried. About 1 mg of plasmid pKC283 was obtained and stored at 4°C in TE buffer at a concentration of about 1 $\mu$g/$\mu$l. A restriction site and function map of plasmid pKC283 is presented in Figure 1 of the accompanying drawings.

Example 2

Construction of Plasmid pKC283PX

About 10 $\mu$l of the plasmid pKC283 DNA prepared in Example 1 were mixed with 20 $\mu$l 10 X medium-salt restriction buffer (500 mM NaCl; 100 mM Tris-HC1, pH 7.5; 100 mM MgCl$_2$; and 10 mM DTT), 20 $\mu$l 1 mg/ml BSA, 5 $\mu$l restriction enzyme Pvu II (~50 Units, as defined by Bethesda Research Laboratories (BRL), from which all restriction enzymes used herein were obtained), and 145 $\mu$l of water, and the resulting reaction was incubated at 37°C for 2 hours. Restriction enzyme reactions described herein were routinely terminated by phenol and then chloroform extractions, which were followed by precipitation of the DNA, an ethanol wash, and resuspension of the DNA in TE buffer. After terminating the Pvu II digestion as described above, the Pvu II-digested plasmid pKC283 DNA was precipitated and then resuspended in 5 $\mu$l of TE buffer.

About 600 picomoles of Xho I linkers (5'-CCTCGAGG-3') were kinased in a mixture containing 10 $\mu$l 5 X Kinase Buffer (300 mM Tris-HC1, pH 7.8; 50 mM MgCl$_2$; and 25 mM DTT), 5 $\mu$l 5 mM ATP, 24 $\mu$l H$_2$0, 0.5 $\mu$l of T4 polynucleotide kinase (about 2.5 units as defined by P-L Biochemicals), 5 $\mu$l 1 mg/ml BSA, and 5 $\mu$l of 10 mM spermidine by incubating the mixture at 37°C for minutes.

About 12.5 $\mu$l of the kinased Xho I linkers were added to the 5 $\mu$l of Pvu II-digested plasmid pKC283 DNA, and then 2.5 $\mu$l of 10 X ligase buffer (300 mM Tris-HC1, pH 7.6; 100 mM MgCl$_2$; and 50 mM DTT), 2.5 $\mu$l of 1 mg/ml BSA, 7 $\mu$l of 5 mM ATP, 2.5 $\mu$l (about 2.5 units as defined by P-L Biochemicals) of T4 DNA ligase, 2.5 $\mu$l of 10 mM spermidine, and 3 $\mu$l of water were added to the DNA. The resulting ligation reaction was incubated at 4°C overnight. After the ligation reaction, the reaction mixture was adjusted to have the composition of high-salt buffer (0.1 M NaCl; 0.05 M Tris-HC1, pH 7.5; 10.0 mM MgCl$_2$; and 1 mM DTT). About 10 $\mu$l (100 units) of restriction enzyme Xho I were added to the mixture, and the resulting reaction was incubated at 37°C for 2 hours.

The reaction was terminated, and the Xho I-digested DNA was precipitated, resuspended, and ligated as described above, except that no Xho I linkers were added to the ligation mixture. The ligated DNA constituted the desired plasmid pKC283PX. A restriction site and function map of plasmid pKC283PX is presented in Figure 2 of the accompanying drawings.

Example 3

## Construction of E. coli K12 MO(λ⁺)/pKC283PX

E. coli K12 MO(λ⁺)can be obtained from the Northern Regional Research Laboratories in lyophylized form under the accession number NRRL B-15993. E. coli K12 MO(λ⁺) comprises the wild-type lambda pL cI repressor gene, so that transcription from the hybrid pL-lpp promoter of the present invention does not occur in E. coli K12 MO(λ⁺) cells. The lyophils are reconstituted, single colonies of MO(λ⁺) are isolated, and a 10 ml overnight culture of the MO(λ⁺)cells is prepared in substantial accordance with the procedure of Example 1, except that the temperature of incubation is 37°C and no ampicillin is used in the growth media.

Fifty $\mu$l of the overnight culture were used to inoculate 5 ml of LB media which also contained 10 mM MgSO₄ and 10 mM MgCl₂. The culture was incubated at 37°C overnight with vigorous shaking. The following morning, the culture was diluted to 200 ml with LB media containing 10 mM MgSO₄ and 10 mM MgCl₂. The diluted culture was incubated at 37°C with vigorous shaking until the absorbance at 550 nm (A₅₅₀) was about 0.5, which indicated a cell density of about 1 x 10⁸ cells/ml. The culture was cooled for ten minutes in an ice-water bath, and the cells were then collected by centrifugation at 4000 g for 10 minutes at 4°C. The cell pellet was resuspended in 100 ml of cold 10 mM MgSO₄ and then immediately re-pelleted by centrifugation. The cell pellet was resuspended in 100 ml of 30 mM CaCl₂ and incubated on ice for 20 minutes.

The cells were again collected by centrifugation and resuspended in 10 ml of 30 mM CaCl₂. A one-half ml aliquot of the cells was added to the ligated DNA prepared in Example 2; the DNA had been made 30 mM in CaCl₂. The cell-DNA mixture was incubated on ice for one hour, heat-shocked at 42°C for 90 seconds, and then chilled on ice for about two minutes. The cell-DNA mixture was diluted into 10 ml of LB media in 125 ml flasks and incubated at 37°C for one hour. One hundred $\mu$l aliquots were plated on LB-agar plates containing ampicillin and incubated at 37°C until colonies appeared.

The colonies were individually cultured, and the plasmid DNA of the individual colonies was examined by restriction enzyme analysis and gel electrophoresis. Plasmid DNA isolation was performed on a smaller scale in accordance with the procedure of Example 1, but the CsC1 gradient step was omitted until the desired E. coli K12 MO(λ⁺)/pKC283PX transformants were identified. A restriction site and function map of plasmid pKC283PX is presented in Figure 2 of the accompanying drawings.

Example 4

## Construction of E. coli K12 MO(λ⁺)/pKC283-L

Ten $\mu$g of plasmid pKC283PX DNA prepared in accordance with the procedure of Example 1 were dissolved in 20 $\mu$l of 10X high-salt buffer, 20 $\mu$l 1 mg/ml BSA, 5 $\mu$l (~50 units) restriction enzyme Bgl II, 5$\mu$l (~50 units) restriction enzyme Xho I, and 150 $\mu$l of water, and the resulting reaction was incubated at 37°C for two hours. The reaction was stopped, and after precipitating the Bgl II-Xho I digested DNA, the DNA was resuspended in 5 $\mu$l of TE buffer.

A DNA linker with single-stranded DNA ends characteristic of Bgl II and Xho I restriction enzyme cleavage was synthesized and kinased. The linker was kinased in substantial accordance with the procedure of Example 2. The DNA linker had the following structure:

$$5'-GATCTATTAACTCAATCTAGAC-3'$$
$$||||||||||||||||||||$$
$$3'-ATAATTGAGTTAGATCTGAGCT-5'$$

The linker depicted above was synthesized from single-stranded deoxyoligonucleotides by procedures well known in the art. The single-stranded deoxyoligonucleotides can be synthesized with commercially available instruments, such as the 380A DNA Synthesizer marketed by Applied Biosystems (850 Lincoln Centre Drive, Foster City, CA 94404), which utilizes phosphoramidite chemistry. Other procedures for synthesizing DNA are also known in the art. The conventional modified phosphotriester method of synthesizing single

13

stranded DNA is described in Itakura et al ., 1977, Science 198:1056 and in Crea et al ., 1978, Proc. Nat. Acad. Sci. USA 75:5765. In addition, an especially preferred method of synthesizing DNA is disclosed in Hsiung et al ., 1983, Nucleic Acid Research 11:3227 and Narang et al ., 1980, Methods in Enzymology 68:90.

The linker and Bgl II-Xho I-digested plasmid pKC283PX were ligated in substantial accordance with the procedure of Example 2. The ligated DNA constituted the desired plasmid pKC283-L. A restriction site and function map of plasmid pKC283-L is presented in Figure 3 of the accompanying drawings. The plasmid pKC283-L DNA was used to transform E. coli K12 MO($\lambda^+$) and the resulting E. coli K12 MO($\lambda^+$)/pKC283-L transformants were identified in substantial accordance with the procedure of Example 3.

## Example 5

### Construction of E. coli K12 MO($\lambda^+$)/pKC283-LB

About 10 μg of plasmid pKC283-L DNA, prepared in substantial accordance with the procedures of Example 1, were dissolved in 20 μl 10X high-salt buffer, 20 μl 1 mg/ml BSA, 5 μl (~50 units) restriction enzyme Xho I, and 155 μl of H₂0, and the resulting reaction was incubated at 37° C for two hours. The Xho I-digested plasmid pKC283-L DNA was then precipitated from the reaction mixture by the addition of three volumes of 95% ethanol and one-tenth volume of 3 M sodium acetate, incubation in a dry ice-ethanol bath for five minutes, and centrifugation. The resulting DNA pellet was washed with 70% ethanol, dried, and resuspended in 2 μl 10X nick-translation buffer (0.5 M Tris-HC1, pH 7.2; 0.1 M MgSO₄; and 1 mM DTT), 1 μl of a solution 2 mM in each of the deoxynucleotide triphosphates, 15 μl of H₂0, 1 μl (~6 units as defined by P-L Biochemicals) of Klenow, which is the large fragment of E. coli DNA polymerase I, and 1 μl of 1 mg/ml BSA. The resulting reaction was incubated at 25° C for 30 minutes; the reaction was stopped by incubating the solution at 70° C for five minutes.

Bam HI linkers (5'-CGGGATCCCG-3') were kinased and ligated to the Xho I-digested, Klenow-treated plasmid pKC283-L DNA in substantial accordance with the procedure of Example 2. After the ligation reaction, the DNA was digested with about 100 units of Bam HI for about 2 hours at 37° C in high-salt buffer. After the Bam HI digestion, the DNA was prepared for ligation in substantial accordance with the procedure of Example 2.

The ~5.9 kb Bam HI restriction fragment was circularized by ligation and transformed into E. coli K12 MO($\lambda^+$) in substantial accordance with the procedures of Examples 2 and 3. The E. coli K12 MO($\lambda^+$)/ pKC283-LB transformants were identified, and then plasmid pKC283-LB DNA was prepared in substantial accordance with the procedure of Example 1. A restriction site and function map of plasmid pKC283-LB is presented in Figure 4 of the accompanying drawings.

## Example 6

### Construction of E. coli K12 MO($\lambda^+$)/pL32

About 10 μg of plasmid pKC283PX were digested with restriction enzyme Sal I in high-salt buffer, treated with Klenow, and ligated to Eco RI linkers (5'-GAGGAATTCCTC-3') in substantial accordance with the procedure of Example 5, with the exception of the starting plasmid, restriction enzymes, and linkers used. After digestion with restriction enzyme Eco RI, which results in the excision of ~2.1 kb of DNA, the ~4.0 kb Eco RI restriction fragment was circularized by ligation to yield plasmid pKC283PRS. The ligated DNA was used to transform E. coli K12 MO($\lambda^+$) in substantial accordance with the procedure of Example 3. After the E. coli K12 MO($\lambda^+$)/pKC283PRS transformants were identified, plasmid pKC283PRS DNA was prepared in substantial accordance with the procedure of Example 1. A restriction site and function map of plasmid pKC283PRS is presented in Figure 5 of the accompanying drawings.

About 10 μg of plasmid pKC283PRS were digested in 200 μl of high-salt buffer with about 50 units each of restriction enzymes Pst I and Sph I. After incubating the reaction at 37° C for about 2 hours, the reaction mixture was electrophoresed on a 0.6% low-gelling-temperature agarose (FMC Corporation, Marine Colloids Division, Rockland, Maine 04841) gel for 2-3 hours at ~130 V and ~75 mA in Tris-Acetate buffer.

The gel was stained in a dilute solution of ethidium bromide, and the band of DNA constituting the ~0.85 kb Pst I-Sph I restriction fragment, which was visualized with long-wave UV light, was cut from the gel in a small segment. The volume of the segment was determined by weight and density of the segment, and an equal volume of 10 mM Tris-HCl, pH 7.6, was added to the tube containing the segment. The segment was then melted by incubation at 72° C. About 1 ug of the ~0.85 kb Pst I-Sph I restriction fragment of plasmid pKC283PRS was obtained in a volume of about 100 μl. In an analogous manner, plasmid pKC283-LB was digested with restriction enzymes Pst I and Sph I, and the resulting ~3.0 kb restriction fragment was isolated by agarose gel electrophoresis and prepared for ligation.

The ~0.85 kb Pst I-Sph I restriction fragment of plasmid pKC283PRS was ligated to the ~3.0 kb Pst I-Sph I restriction fragment of plasmid pKC283-LB in substantial accordance with the procedure of Example 2. The ligated DNA constituted the desired plasmid pL32. A restriction site and function map of plasmid pL32 is presented in Figure 6 of the accompanying drawings. Plasmid pL32 was transformed into E. coli K12 MO(λ⁺) in substantial accordance with the procedure of Example 3. Plasmid pL32 DNA was prepared from the E. coli K12 MO(λ⁺)/pL32 transformants in substantial accordance with the procedure of Example 1. Analysis of the plasmid pL32 DNA demonstrated that more than one Eco RI linker attached to the Klenow-treated, Sal I ends of plasmid pKC283PX. The presence of more than one Eco RI linker does not affect the utility of plasmid pL32 or derivatives of plasmid pL32 and can be detected by the presence of an Xho I restriction site, which is generated whenever two of the Eco RI linkers are ligated together. Alternatively, plasmid pL32 may be constructed by carrying out the Sal I-Eco RI excision and ligation of the first paragraph of this Example upon plasmid pKC283-LB.

## Example 7

## Construction of E. coli K12 MO(λ⁺)/pL47

E. coli K12 RV308/pNM789 can be obtained from the Northern Regional Research Laboratories in lyophilized form under the accession number NRRL B-18216. A restriction site and function map of pNM789 is presented in Figure 7 of the accompanying drawings. Plasmid DNA is extracted from the culture in substantial accordance with the teaching of Example 1, except that the temperature of incubation is 37° C. Ten micrograms of pNM789 are suspended in 200 μl Pvu II buffer (50 mM Tris-HCl (pH 7.5), 60 mM NaCl and 6mM MgCl₂). One unit of Pvu II is added and the reaction mix is incubated for 5 minutes at 37° C. The enzyme is inactivated by heating 10 minutes at 65° C. 30 μl of 10X Bam HI buffer (200 mM Tris-HCl (pH 8.0), 1M NaCl and 70 mM MgCl₂), 70 μl H₂O and 10 units of Bam HI are next added and the reaction is incubated for 1 hour at 37° C. This is followed by the addition of 5 units of alkaline phosphatase and incubation for 1 hour at 65° C. The DNA fragments are separated on a 1 percent agarose gel, and a DNA fragment (Figure 3) the size of a single cut fragment is purified.

A DNA linker with a blunt end and a Bam HI end is synthesized in substantial accordance with the teaching of Example 4. This linker (shown in Figure 3) has the following structure:

$$5'\text{-CTGTGCCTTCTAG-3'}$$
$$|\,|\,|\,|\,|\,|\,|\,|\,|\,|\,|\,|\,|$$
$$3'\text{-GACACGGAAGATCCTAG-5'}$$

The linker is kinased and ligated into the Bam HI-Pvu II digested plasmid pNM789 in substantial accordance with the teaching of Example 2. This ligation mixture is used to transform E coli K12 RV308 cells and plasmid isolation is performed upon these transformants in substantial accordance with the teaching of Example 3. Several plasmids are selected which contain the appropriate size Pvu II fragment (494bp) and Xba I-Bam HI fragment (628bp). The sequence of at least two of these is determined by sequencing from the Bam HI site toward the unique Sma I site and one clone is selected with the desired sequence. This intermediate plasmid is designated plasmid 120. A schematic outline of this procedure and a restriction site and function map of plasmid 120 is presented in Figure 8 of the accompanying drawings.

To isolate the EK-BGH-encoding DNA, about 10 μg of plasmid 120 were digested in 200 μl of high-salt buffer containing about 50 units each of restriction enzymes Xba I and Bam HI. The digestion products were separated by agarose gel electrophoresis, and the ~0.6 kb Xba I-Bam HI restriction fragment which encodes EK-BGH was isolated and prepared for ligation in substantial accordance with the procedure of Example 6.

Plasmid pL32 was also digested with restriction enzymes Xba I and Bam HI, and the ~3.9 kb restriction fragment was isolated and prepared for ligation. The ~3.9 kb Xba I-Bam HI restriction fragment of plasmid pL32 was ligated to the ~0.6 kb Xba I-Bam HI restriction fragment of plasmid 120 in substantial accordance with the procedure of Example 2 to yield plasmid pL47. A restriction site and function map of plasmid pL47 is presented in Figure 9 of the accompanying drawings. Plasmid pL47 was transformed into E. coli K12 MO($\lambda^+$) in substantial accordance with the procedure of Example 3, and the E. coli K12 MO($\lambda^+$)/pL47 transformants were identified. Plasmid pL47 DNA was prepared from the transformants in substantial accordance with the procedures of Example 1.

## Example 8

## Construction of pBR322-IT

The synthetic gene encoding AaIT was created from the amino acid sequence of the insecticidal neurotoxin of scorpion Androctonus australis (Zlotkin, E. 1983. Insect Biochem . 13 , 219-236) using techniques well known in the art. The DNA fragment contained the substance coding for the AaIT amino acids +1 through +69, the positive strand sequence of which is:


    5'-GAT  CCAAATAATG  AAAAAAAACG  GCTACGCTGT  TGACTCTTCT

    GGCAAAGCTC  CGGAATGCCT  GCTGTCTAAC  TACTGCAACA  ACCAGTGCAC

    TAAAGTTCAT  TACGCTGACA  AAGGCTACTG  CTGCCTGCTG  TCCTGCTACT

    GCTTCGGCCT  GAACGACGAC  AAAAAAGTTC  TGGAAATCTC  TGACACTCGT

    AAATCTTACT  GCGACACTAC  TATCAACTAA  TAG-3'


The synthetic AaIT gene contained recognition sequences for the restriction endonuclease Bam HI at both the 5' and 3' ends in order to facilitate proper insertion into plasmid pBR322. Plasmid pBR322 (New England Biolabs, 32 Tozer Road, Beverly, MA 01915) was digested with endonuclease Bam HI. After phosphorylation with T4 polynucleotide kinase (Pharmacia-LKB Biotechnology, 800 Centennial Avenue, Piscataway, NJ 08854), the synthetic AaIT gene was cloned into Bam HI-digested plasmid pBR322 to create plasmid pBR322-IT in substantial accordance with the teaching of Example 3.

## Example 9

## Construction of Plasmid pRB-IT

In order to add the DNA sequence encoding the signal peptide of human interleukin-2 immediately upstream of and in proper reading frame with the AaIT gene, the endonuclease Hin F1 cleavage site at the position corresponding to AaIT amino acids +8 and +9 was used to join synthetic DNA segment A. DNA Segment A contained the substance encoding amino acid residues -20 to -1 of the signal peptide of human interleukin-2 and the first 8 amino acid residues of AaIT, the positive strand sequence of which is:

```
GAT  CTA  TAA  ATA  ATG  TAC  AGG  ATG  CAA  CTC
CTG  TCT  TGC  ATT  GCA  CTA  AGT  CTT  GCA  CTT
GTC  ACA  AAC  AGT  AAA  AAA  AAC  GGC  TAC  GCT
GTT  G
```

DNA Segment A contained recognition sequences for endonucleases Bgl II and Hin F1 at the 5′ end and 3′ end respectively and was synthesized using standard techniques according to the teachings of Brown, E.L., Belagaje, R., Ryan, M.J., and Khorana, H. G., (1979), Methods in Enzymology 68 :109-151 (Wu, R. ed.) Academic Press, NY, the entire teaching of which is herein incorporated by reference.

Approximately 10 μl of the plasmid pBR322-IT prepared above was mixed with 20μl of the 10x high salt restriction buffer (Example 2), 20 μl of 1 mg/ml BSA, 5μl of Bam H1, 5μl of Hin F1, 75 μl of $H_2O$. The reaction was incubated at 37° C for 2 hours. The Bam HI digested DNA was then precipitated with ethanol and 3M NaOAC, and purified by electrophoresis on a 1% low-melting agarose gel. The smaller fragment was sliced from the gel and the DNA was isolated by the Elutip-d column procedure (Schleicher and Schuell, Keene, N.H.). After precipitation and centrifugation, the DNA pellet was resuspended in 10 μl of 10 x Hin F1 restriction buffer (IM NaCl, 60 mM Tris-HCl pH 7.4, 60 mM $MgCl_2$, 60 mM 2-mercaptoethanol, 10 μl of 1 mg/ml BSA). The Hin F1 to Bam H1 sequence (Segment B) was separated by electrophoresis in a 1% low-melt agarose gel. The larger fragment corresponding to Segment B was sliced from the gel by the Elutip-d column procedure. After precipitation and drying, the DNA was stored in 20 μl of 10 mM Tris-HCl pH 8.0. DNA Segment B contained the substance encoding AaIT amino acids +9 to +69, the positive strand sequence of which is:

```
   AC  TCT  TCT  GGC  AAA  GCT  CCG  GAA  TGC
CTG  CTG  TCT  AAC  TAC  TGC  AAC  AAC  CAG  TGC
ACT  AAA  GTT  CAT  TAC  GCT  GAC  AAA  GGC  TAC
TGC  TGC  CTG  CTG  TCT  TGC  TAC  TGC  TTC  GGC
CTG  AAC  GAC  GAC  AAA  AAA  GTT  CTG  GAA  ATC
TCT  GAC  ACT  CGT  AAA  TCT  TAC  TGC  GAC  ACT
ACT  ATC  AAC  TAA  TAG
```

Segment A and Segment B when ligated properly through joining at the Hin F1 site generated a chimeric gene, SP-IT, encoding amino acids -1 to -20 of human interleukin-2 signal peptide and amino acids +1 to +69 of AaIT, with a Bgl II site at the 5′ end and Bam HI site at 3′ end. The positive strand sequence of hybrid SP-IT gene is:

```
5' - GAT  CTA  TAA  ATA  ATG  TAC  AGG  ATG  CAA  CTC
                         Met  Tyr  Arg  Met  Gln  Leu

      CTG  TCT  TGC  ATT  GCA  CTA  AGT  CTT  GCA  CTT
      Leu  Ser  Lys  Ile  Ala  Leu  Ser  Leu  Ala  Leu

      GTC  ACA  AAC  AGT  AAA  AAA  AAC  GGC  TAC  GCT
      Val  Thr  Asn  Ser  Lys  Lys  Asn  Gly  Tyr  Ala

      GTT  GAC  TCT  TCT  GGC  AAA  GCT  CCG  GAA  TGC
      Val  Asp  Ser  Ser  Gly  Lys  Ala  Pro  Glu  Cys

      CTG  CTG  TCT  AAC  TAC  TGC  AAC  AAC  CAG  TGC
      Leu  Leu  Ser  Asn  Tyr  Cys  Asn  Asn  Gln  Cys

      ACT  AAA  GTT  CAT  TAC  GCT  GAC  AAA  GGC  TAC
      Thr  Lys  Val  His  Tyr  Ala  Asp  Lys  Gly  Tyr

      TCG  TGC  CTG  CTG  TCT  TGC  TAC  TGC  TTC  GGC
      Cys  Cys  Leu  Leu  Ser  Cys  Tyr  Cys  Phe  Gly

      CTG  AAC  GAC  GAC  AAA  AAA  GTT  CTG  GAA  ATC
      Leu  Asn  Asp  Asp  Lys  Lys  Val  Leu  Glu  Ile

      TCT  GAC  ACT  CGT  AAA  TCT  TAC  TGC  GAC  ACT
      Ser  Asp  Thr  Arg  Lys  Ser  Tyr  Cys  Asp  Thr

      ACT  ATC  AAC  TAA  TAG  -  3'
      Thr  Ile  Asn  *    *
```

About 10 μl of the plasmid pL47 prepared in Example 7 was mixed with 20 μl of medium salt restriction buffer, 20 μl of 1 mg/ml BSA, 10 units of restriction enzymes Bgl II and Bam HI, and water to create a final volume of 200 μl. The mixture was incubated at 37° C for two hours and the DNA precipitated with 1 ml EtOH and 20 μl 3M NaOAc. The DNA was purified by electrophoresis on a 1% low-melting agarose gel. The larger fragment was sliced from the gel and the DNA recovered by the elutip-d procedure. After precipitation and drying the DNA pellet was dissolved in 70 μl of 10 x CIAP buffer (50 mM Tris-HCl, pH 8.0, 10 mM MgCl₂, 1 mM ZnCl₂), 85 μl of water and 5 μl of calf-intestine alkaline phosphatase (Boehringer-Mannheim Biochemicals, P.O. Box 50414, Indianapolis, IN 46250). The reaction was incubated at 37° C for 1 hour and then extracted with 100 μl Phenol/Chloroform (1:1, v/v). The DNA was precipitated by making the reaction mixture 0.3 M NaOAc, adding 3 volumes of ethanol, mixing and chilling to -70° C and centrifuging. The DNA pellet was dissolved in 20 μl of 10 mM Tris-HCl (pH 8.0), 1 mM EDTA, and purified by electrophoresis on a 1% low melting agarose gel. The DNA band was cut from the gel and the DNA isolated by the Elutip-d procedure. After precipitation and drying the DNA was stored in 20 μl of 10 mM Tris-HCl (pH 8.0). This DNA represents the approximately 3.9 kb Bgl II to Bam HI fragment corresponding to the vector portion of plasmid pL 47. This vector DNA was ligated with DNA segments A and B to form the pRB-IT plasmid. Transfection, amplification and isolation of the plasmid was performed in accordance with the teaching of Example 3. A restriction site and function map of plasmid pRB-IT is presented in Figure 10 of the accompanying drawings. The sequence of the SP-IT gene is presented in Figure 11 of the accompanying drawings.

Example 10

18

## Construction of the pMSV-IT Plasmid

The pRB-IT plasmid was isolated by cesium chloride-ethdium bromide density gradient centrifugation as outlined in Example 1, then digested with Bgl II and BamH1 endonucleases according to the procedure of Example 10. The 285 bp fragment encoding the SP-IT gene was isolated by agarose gel electrophoresis and solubilized from the gel material. The pMSV plasmid (NRRL B-15929) was amplified in E. coli K12 HB101 (NRRL B-15626) and isolated in substantial accord with the procedure of Example 3.

Approximately 10 $\mu$l of the pMSV plasmid was mixed with 20 $\mu$l of 10x medium salt restriction buffer, 20 $\mu$l 1 mg/ml BSA, 5 $\mu$l of Bgl II restriction endonuclease, and 145 $\mu$l of water and incubated at 37$^\circ$C for two hours. The restriction enzyme reaction was terminated by phenol/chloroform extractions. The DNA was precipitated with ETOH followed by wash in 70% ETOH and resuspension in 5 $\mu$l of TE buffer.

Approximately 10 $\mu$l of the linearized SP-IT (285 bp) fragment was added to the Bgl II digested pMSV plasmid. About 2.5 $\mu$l of 10x ligase buffer 2.5 $\mu$l of 1 mg 1 ml BSA, 7 $\mu$l of 5 mm ATP, 2.5 units of T4 DNA ligase, 2.5 $\mu$l of 10 mm spermidine and 5 $\mu$l of water was added and the reaction was incubated overnight at 4$^\circ$C.

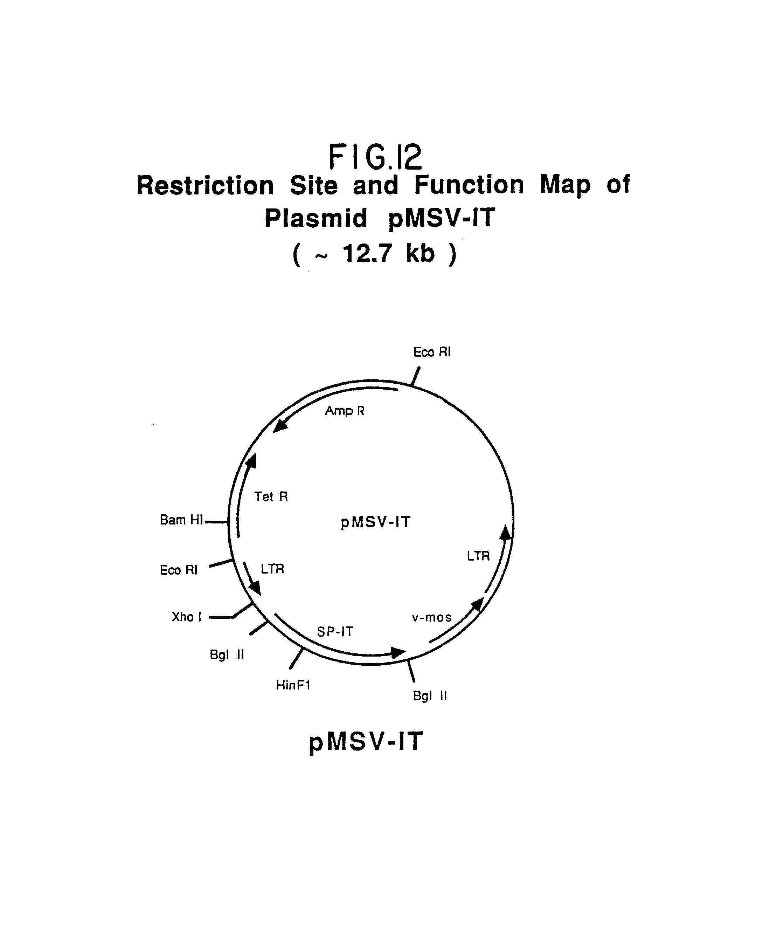

The pMSV-IT plasmid created by the fusion of the SP-IT gene into the pMSV plasmid was directly transformed into the E. Coli K12 HB101 (NRRL B-15626) and the plasmid amplified and isolated substantially in accord with Example 3. A restriction site and function map of plasmid pMSV-IT is presented in Figure 12 of the accompanying drawings.

## Example 11

### Transfection of NIH/3T3 Cells with pMSV and pMSV-IT

Approximately 0.5 x 10$^5$ NIH/3T3 mouse fibroblast cells (ATCC CCL92) suspended in 3 ml of DMEM containing 10% fetal calf serum (EF10) were plated onto 3.5 cm tissue culture dish. The cultures were maintained in a humidified $CO_2$ incubator at 37$^\circ$C overnight. 10 $\mu$l of the CsCl density gradient purified plasmids pMSV and pMSV-IT were resuspended in separate tubes in 450 $\mu$l of HBS (20 mM Hepes 137 mM NaCl, 5 mM KCl, 0.7 mM $Na_2HPO_4$, 6 mM dextrose, final pH7.0). 30 $\mu$l of 2 M $CaCl_2$ were added to each tube to obtain a final [$CaCl_2$] = 125 mM. The tubes were then incubated at room temperature 20-30 minutes.

While the DNA was being precipitated, the NIH/3T3 cells were washed with phosphate buffered saline (PBS) containing 100 units/ml of penicillin and 100 $\mu$g/ml of streptomycin and refed with 1 ml of EF10 media (Dulbecco minimal media + 10% fetal calf serum). The DNA precipitates were added to the cell cuture. The plates were swirled gently to distribute DNA evenly. The cells were then incubated at 37$^\circ$C in a $CO_2$-incubator for 5 hours. After removal of media, the cells were washed once with PBS, followed by brief treatment (45-60 seconds) of 25% DMSO in PBS. After DMSO shock treatment, the cells were washed once gently with pBS, refed with 2 ml of EF10 and returned to $CO_2$-incubator. The appearance of cells transformed by v-mos was checked microscopically daily. When the foci containing v-mos -transformed cells were well established and visible by naked eyes, they were picked and transferred to 96-well microtiter plates to established individual transformed clones. When the clones reached confluency, they were trypsinized and expanded into larger cultures. Several cell lines were established from NIH/3T3 cells transformed by pMSV-IT and by the parental plasmid pMSV.

Northern blot analysis of RNA was used to identify high-producer lines of AaIT. Approximately 5x10$^6$ cells grown to 75-80% confluency were used to prepare RNA. RNA preparations were made from NIH/3T3, DC-1 and DB-2 cells by guanidium isothiocyanate procedure developed by Chirgwin et al . Twenty micrograms of RNA from each sample were heat-treated with glyoxal and applied to 1.25% formaldehyde/agarose gel. After electrophoresis, RNA was transferred to nitrocellulose paper and hybridized with $^{32}$p-labeled Bgl II - Bam HI fragment containing SP-IT sequence isolated from plasmid pRB-IT. The probe was labeled by nick translation with BRL nick translation kit. Autoradiography was carried out with Kodak XAR-5-films and DuPont Cronex intensifying screens.

Preliminary dot-blot hybridization analysis of cytoplasmic RNA from these cell lines indicated that all of them contained v-mos related transcripts, while only those transformed by pMSV-IT expressed AaIT-related RNA transcripts. Total RNA prepared by guanidium isothiocyanate procedure was separated in formaldehyde/agarose gel, transferred to nitrocellulose paper paper and hybridized with $^{32}$P-labeled Bgl II - Bam HI fragment containing the chimeric SP-IT gene. In cells transformed by pMSV-IT, the expression of SP-IT sequence driven by LTR and initiated at the R region of LTR should yield a genome-length transcript 5,528 bases long. As expected, DC-1 and DC-1A cells transformed by pMSV-IT synthesized an AaIT-hybridizing RNA transcript approximately 5.7 kilobases long. In contrast, neither NIH/3T3 cells nor DB-2 cells transformed by parental plasmid pMSV produced any RNA transcripts that hybridized to the SP-IT probe.

To test whether any biologically active rAaIT was secreted into culture medium by pMSV-IT transformed cells, DC-1 cells which appeared to synthesize the highest level of AaIT-containing transcript and DB-2 cells transformed by pMSV were expanded into mass cultures (~4-5 x 10$^8$ cells each). When the cultures reached 75-80% confluency, the regular serum-containing DME was replaced by serum-free DME. Twenty-four hours later, the conditioned media were collected and the process was repeated once. The combined culture fluids were cleared of cell debris by low-speed centrifugation, followed by ultrafiltration with an Amicon concentrator fitted with YM 10 membrane filter. Aliquots were collected when the cultrue fluids were concentrated approximately 5-fold, 20-fold, 50-fold, 100-fold, 200-fold and 300-fold. The concentrates were used directly for toxicity studies soon after the concentration process was completed.

## Example 12

## Mosquito Larvicide Screening Assay

The effectiveness of the secreted insect toxin was verified by the ability of the culture media containing the secreted toxin to kill mosquito instar larvae. Yellow fever mosquitos ( Aedes aegypti ) were given a blood meal after which eggs were laid on wet paper towels. The eggs were then transferred to a container of hatching water which was prepared from one liter of sterilized distilled water containing small amounts (<5 mg) of yeast and finely ground Purina laboratory chow. The eggs hatched rapidly (10-15 min). Larvae were refed with 5 mg of finely ground Purina laboratory chow on day 2. After approximately three days, the mosquito reached the second instar larval stage which was ideal for testing.

Equal volumes (50 microliters) of hatching water containing 10-15 larvae and tissue culture concentrates from pMSV-IT transformed 3T3 (DC-1) cells were placed in the well of 96 well microtiter plate. This procedure was repeated for each concentration of culture media prepared in Example 11. Mortality is recorded at 2 hours, 4 hours, and 24 hours after exposure to the culture medium. Table 1 is representative of the data obtained from these assay procedures. NIH/3T3 cells transformed by pMSV were used as a control with respect to the pMSV-IT transformed (DB-2) cells.

It is evident from the data shown in Table 1 that the culture media of the pMSV-IT transformed cells contained insect toxin which was not apparent in the control sample. The data also demonstrate the concentration-dependent effectiveness of the insect toxin. This data demontrates that the NIH/3T3 cells transformed with the pMSV-IT plasmid containing the SP-IT gene secreted a functional insect toxin protein into the culture media.

## Example 13

## Toxicity Study in Mice

In order to ascertain that the proper toxin gene was isolated and that it was non-toxic to mammals, mice were injected with concentrates of the culture broth of pMSV-IT transformed 3T3 (DC-1) cells and pMSV transformed 3T3 (DB-2) cells as well as the crude scorpion venom (Sigma, P.O. Box 14508, St. Louis, MO 63178). The study was performed with the 300x concentrates of the culture broth. The AaIT in the 300x concentrate of pMSV-IT-3T3 culture broth was estimated to be 15 μg/ml. Approximately 0.5 ml of each concentrate was intravenously injected into each of 3 ICR female mice (Charles River Laboratories, Inc., 251 Ballardvale Street, Wilmington, MA 01887). The dose of AaIT administered was approximately 0.3-0.4 grams/gram of body weight. A third group of three mice were injected with 0.5 ml of 15 $\mu$g/ml crude scorpion venom. 15$\mu$g/ml is approximately twice the $LD_{50}$ of scorpion venom reported. Zlotkin, E. et al . (1971) Biochimie 53 , 1073-1078. The mice were observed hourly on the day of injection and daily thereafter for two weeks for mortality signs of toxicity such as hypoactivity, lethargy, comatose, hind leg paralysis, tremor, and diarrhea.

**Claims**

1. A method for producing a functional secreted insect toxin in a recombinant eucaryotic host cell, said method comprising:

A. transforming or transfecting said eucaryotic host cell with a recombinant DNA cloning vector comprising:

i. an origin of replication or integrating sequence,

ii. a promoter or translational activating sequence functional in said host cell,

iii. a DNA sequence encoding a mammalian signal peptide,

iv. a DNA sequence encoding an insect toxin gene, and,

v. a selectable marker, then

B. culturing said eucaryotic host cell under conditions suitable for the expression and secretion of said functional insect toxin.

2. The method of Claim 1 wherein said DNA sequence encoding a mammalian signal peptide is selected from the group consisting of the DNA sequence encoding the human insulin signal peptide, the DNA sequence encoding the rat insulin I signal peptide, the DNA sequence encoding the rat insulin II signal peptide, the DNA sequence encoding the rat growth hormone signal peptide, the DNA sequence encoding the human growth hormone signal peptide, and the DNA sequence encoding the human interleukin 2 signal peptide.

3. The method of Claim 2 wherein the DNA sequence encoding a mammalian signal peptide is the DNA sequence encoding the human interleukin 2 signal peptide.

4. The method of any of Claim 1, 2, or 3, wherein the DNA sequence encoding an insect toxin gene is selected from the group consisting of the DNA sequence encoding the AaIT toxin gene, a DNA sequence encoding a functional derivative of the insect toxin gene, and the DNA sequence:

AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

5. The method of Claim 4, wherein said DNA sequence encoding an insect toxin gene is the DNA sequence encoding the AaIT insect toxin gene.

6. The method of Claim 4 wherein said DNA sequence encoding an insect toxin gene is a DNA sequence encoding a functional derivative of the AaIT insect toxin gene.

7. The method of Claim 4 wherein said DNA sequence is:

AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

8. The method of Claim 1 wherein said eucaryotic host cell is selected from the group consisting of AV12 cells, HeLa cells, BHK 21 cells, 293 cells, and 3T3 cells.

9. The method of Claim 8 wherein said eucaryotic host cell is a 3T3 cell.

10. The method of Claim 9 wherein said selectable marker is selected from the group consisting of the DNA sequence encoding the v-mos gene, the DNA sequence encoding neomycin resistance, and the DNA sequence encoding hygromycin resistance.

11. The method of Claim 10 wherein said selectable marker is the DNA sequence encoding the v-mos gene.

12. The method of Claim 3 wherein said DNA sequence encoding an insect toxin gene is the DNA sequence encoding the AaIT insect toxin gene.

44

13. The method of Claim 3 wherein said DNA sequence encoding an insect toxin gene is the DNA sequence encoding a functional derivative of the AaIT insect toxin gene.

14. The method of Claim 3 wherein said DNA sequence encoding an insect toxin gene is the DNA sequence:

```
AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.
```

15. The method of Claim 12, 13 or 14 wherein said eucaryotic host cell is a 3T3 cell.

16. The method of Claim 13, 14 or 15 wherein said selectable marker is v-mos.

17. The method of Claim 13, 14, 15, or 16 wherein said recombinant vector is pMSV-IT.

18. An insect toxin whenever produced by a method according to any one of the Claims 1 to 17.

19. A recombinant DNA compound comprising
    a. a recombinant DNA cloning vector, and
    b. a DNA sequence encoding a SP-IT gene.

20. The recombinant DNA compound of Claim 19, which further comrpises
    c. a promoter or translational activating sequence.

21. The recombinant DNA compound of Claim 20, which further comprises,
    d. a selectable marker

22. The compound of Claim 21, wherein said DNA sequence encoding said SP-IT gene is the DNA sequence encoding the human interleukin 2 signal peptide operably linked to the DNA sequence encoding the AaIT gene.

23. The compound of Claim 21 wherein said DNA sequence encoding an SP-IT gene is the DNA sequence encoding the human interleukin 2 signal peptide operably linked to a functional derivative of the AaIT gene.

24. The compound of Claim 21, wherein said DNA sequence encoding said SP-IT gene is the DNA sequence encoding the human interleukin 2 signal peptide operably linked to the DNA sequence.

```
AAA AAA AAC GGC TAC GCT GTT GAC TCT
TCT GCG AAA GCT CCG GAA TGC CTG CTG
TCT AAC TAR TGC AAC AAC CAG TGC ACT
AAA GTT CAT TAG GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.
```

25. The compound of Claim 24 that is plasmid pMSV-IT.

Claims for the following Contracting State : GR

1. A method for producing a functional secreted insect toxin in a recombinant eucaryotic host cell, said method comprising:

    A. transforming or transfecting said eucaryotic host cell with a recombinant DNA cloning vector comprising:
        i. an origin of replication or integrating sequence,
        ii. a promoter or translational activating sequence functional in said host cell,
        iii. a DNA sequence encoding a mammalian signal peptide,
        iv. a DNA sequence encoding an insect toxin gene, and,
        v. a selectable marker, then
    B. culturing said eucaryotic host cell under conditions suitable for the expression and secretion of said functional insect toxin.

2. The method of Claim 1 wherein said DNA sequence encoding a mammalian signal peptide is selected from the group consisting of the DNA sequence encoding the human insulin signal peptide, the DNA sequence encoding the rat insulin I signal peptide, the DNA sequence encoding the rat insulin II signal peptide, the DNA sequence encoding the rat growth hormone signal peptide, the DNA sequence encoding the human growth hormone signal peptide, and the DNA sequence encoding the human interleukin 2 signal peptide.

3. The method of Claim 2 wherein the DNA sequence encoding a mammalian signal peptide is the DNA sequence encoding the human interleukin 2 signal peptide.

4. The method of any of Claim 1, 2, or 3, wherein the DNA sequence encoding an insect toxin gene is selected from the group consisting of the DNA sequence encoding the AaIT toxin gene, a DNA sequence encoding a functional derivative of the insect toxin gene, and the DNA sequence:

AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

5. The method of Claim 4, wherein said DNA sequence encoding an insect toxin gene is the DNA sequence encoding the AaIT insect toxin gene.

6. The method of Claim 4 wherein said DNA sequence encoding an insect toxin gene is a DNA sequence encoding a functional derivative of the AaIT insect toxin gene.

7. The method of Claim 4 wherein said DNA sequence is:

AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

8. The method of Claim 1 wherein said eucaryotic host cell is selected from the group consisting of AV12 cells, HeLa cells, BHK 21 cells, 293 cells, and 3T3 cells.

9. The method of Claim 8 wherein said eucaryotic host cell is a 3T3 cell.

10. The method of Claim 9 wherein said selectable marker is selected from the group consisting of the DNA sequence encoding the v-mos gene, the DNA sequence encoding neomycin resistance, and the DNA sequence encoding hygromycin resistance.

11. The method of Claim 10 wherein said selectable marker is the DNA sequence encoding the v-mos gene.

12. The method of Claim 3 wherein said DNA sequence encoding an insect toxin gene is the DNA sequence encoding the AaIT insect toxin gene.

13. The method of Claim 3 wherein said DNA sequence encoding an insect toxin gene is the DNA sequence encoding a functional derivative of the AaIT insect toxin gene.

14. The method of Claim 3 wherein said DNA sequence encoding an insect toxin gene is the DNA sequence:

AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

15. The method of Claim 12, 13 or 14 wherein said eucaryotic host cell is a 3T3 cell.

16. The method of Claim 13, 14 or 15 wherein said selectable marker is v-mos.

17. The method of Claim 13, 14, 15, or 16 wherein said recombinant vector is pMSV-IT.

18. An insect toxin whenever produced by a method according to any one of the Claims 1 to 17.

19. A recombinant DNA compound comprising
   a. a recombinant DNA cloning vector, and
   b. a DNA sequence encoding a SP-IT gene.

20. The recombinant DNA compound of Claim 19, which further comrpises
   c. a promoter or translational activating sequence.

21. The recombinant DNA compound of Claim 20, which further comprises,
   d. a selectable marker
22. The compound of Claim 21, wherein said DNA sequence encoding said SP-IT gene is the DNA sequence encoding the human interleukin 2 signal peptide operably linked to the DNA sequence encoding the AaIT gene.
23. The compound of Claim 21 wherein said DNA sequence encoding an SP-IT gene is the DNA sequence encoding the human interleukin 2 signal peptide operably linked to a functional derivative of the AaIT gene.
24. The compound of Claim 21, wherein said DNA sequence encoding said SP-IT gene is the DNA sequence encoding the human interleukin 2 signal peptide operably linked to the DNA sequence.

AAA AAA AAC GGC TAC GCT GTT GAC TCT
TCT GCG AAA GCT CCG GAA TGC CTG CTG
TCT AAC TAR TGC AAC AAC CAG TGC ACT
AAA GTT CAT TAG GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.
25. The compound of Claim 24 that is plasmid pMSV-IT.

Claims for the following Contracting State : ES

1. A method for producing a functional secreted insect toxin in a recombinant eucaryotic host cell, said method comprising:
   A. transforming or transfecting said eucaryotic host cell with a recombinant DNA cloning vector comprising:
      i. an origin of replication or integrating sequence,
      ii. a promoter or translational activating sequence functional in said host cell,
      iii. a DNA sequence encoding a mammalian signal peptide,
      iv. a DNA sequence encoding an insect toxin gene, and,
      v. a selectable marker, then
   B. culturing said eucaryotic host cell under conditions suitable for the expression and secretion of said functional insect toxin.
2. The method of Claim 1 wherein said DNA sequence encoding a mammalian signal peptide is selected from the group consisting of the DNA sequence encoding the human insulin signal peptide, the DNA sequence encoding the rat insulin I signal peptide, the DNA sequence encoding the rat insulin II signal peptide, the DNA sequence encoding the rat growth hormone signal peptide, the DNA sequence encoding the human growth hormone signal peptide, and the DNA sequence encoding the human interleukin 2 signal peptide.
3. The method of Claim 2 wherein the DNA sequence encoding a mammalian signal peptide is the DNA sequence encoding the human interleukin 2 signal peptide.
4. The method of any of Claim 1, 2, or 3, wherein the DNA sequence encoding an insect toxin gene is selected from the group consisting of the DNA sequence encoding the AaIT toxin gene, a DNA sequence encoding a functional derivative of the insect toxin gene, and the DNA sequence:
AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG GAC GAC AAA AAA GTT CTG
GAA ATC TCT GAC ACT CGT AAA TCT TAC
TGC GAC ACT ACT ATC AAC TAA TAG.
5. The method of Claim 4, wherein said DNA sequence encoding an insect toxin gene is the DNA sequence encoding the AaIT insect toxin gene.
6. The method of Claim 4 wherein said DNA sequence encoding an insect toxin gene is a DNA sequence encoding a functional derivative of the AaIT insect toxin gene.
7. The method of Claim 4 wherein said DNA sequence is:
AAA AAA AAC GGC TAC GCT GTT GAC TCT
TCT GCG AAA GCT CCG GAA TGC CTG CTG

TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG GAC GAC AAA AAA GTT CTG
GAA ATC TCT GAC ACT CGT AAA TCT TAC
TGC GAC ACT ACT ATC AAC TAA TAG.

8. The method of Claim 1 wherein said eucaryotic host cell is selected from the group consisting of AV12 cells, HeLa cells, BHK 21 cells, 293 cells, and 3T3 cells.

9. The method of Claim 8 wherein said eucaryotic host cell is a 3T3 cell.

10. The method of Claim 9 wherein said selectable marker is selected from the group consisting of the DNA sequence encoding the v-mos gene, the DNA sequence encoding neomycin resistance, and the DNA sequence encoding hygromycin resistance.

11. The method of Claim 10 wherein said selectable marker is the DNA sequence encoding the v-mos gene.

12. The method of Claim 3 wherein said DNA sequence encoding an insect toxin gene is the DNA sequence encoding the AaIT insect toxin gene.

13. The method of Claim 3 wherein said DNA sequence encoding an insect toxin gene is the DNA sequence encoding a functional derivative of the AaIT insect toxin gene.

14. The method of Claim 3 wherein said DNA sequence encoding an insect toxin gene is the DNA sequence: AAA AAA AAC GGC TAC GCT GTT GAC TCT
TCT GCG AAA GCT CCG GAA TGC CTG CTG
TCT AAC TAC TGC AAC AAC CAG TGC ACT
AAA GTT CAT TAC GCT GAC AAA GGC TAC
TGC TGC CTG CTG TCT TGC TAC TGC TTC
GGC CTG AAC GAC GAC AAA AAA GTT CTG
GAA ATC TCT GAC ACT CGT AAA TCT TAC
TGC GAC ACT ACT ATC AAC TAA TAG.

15. The method of Claim 12, 13 or 14 wherein said eucaryotic host cell is a 3T3 cell.

16. The method of Claim 13, 14 or 15 wherein said selectable marker is v-mos.

17. The method of Claim 13, 14, 15, or 16 wherein said recombinant vector is pMSV-IT.

# FIG. I
# Restriction Site and Function Map of
# Plasmid pKC283
# (~9.1 kb)

# FIG.2
## Restriction Site and Function Map of Plasmid pKC283PX
## (~6.1 kb)

# FIG.3
## Restriction Site and Function Map of Plasmid pKC283-L
## (~5.9 kb)

# FIG.4
## Restriction Site and Function Map of Plasmid pKC283-LB
## (~5.9 kb)

# FIG.5
## Restriction Site and Function Map of Plasmid pKC283PRS
## (~4.0 kb)

# FIG. 6
## Restriction Site and Function Map of Plasmid pL32
## (~3.9 kb)

# FIG.7
## Restriction Site and Function Map of Plasmid pNM789

# FIG.8

Plasmid pNM789

Pvu II Partial Digestion

Bam HI

Alkaline Phosphatase

Pvu II    Bam HI

Synthetic Fragment

T₄ DNA Ligase

Hind III
Ippp
Xba I
ApR
Pvu II
Pvu II
Bam HI
3'Ipp
Sal I

Hind III
Ippp
Xba I
ApR
Pvu II
Coding Sequence for Bovine Growth Hormone
Pvu II
Bam HI
3'Ipp
Sal I

Plasmid 120

# FIG.9
## Restriction Site and Function Map of
## Plasmid pL47
## (~4.5 kb)

# FIG.IO
## Restriction Site and Function Map of
## Plasmid pRB-IT
## ( ~ 4.8 kb )

**pRB-IT**

# FIG.11

## Sequence of SP-IT

```
5' - GAT   CTA   TAA   ATA   ATG   TAC   AGG   ATG   CAA   CTC
                             Met   Tyr   Arg   Met   Gln   Leu

      CTG   TCT   TGC   ATT   GCA   CTA   AGT   CTT   GCA   CTT
      Leu   Ser   Lys   Ile   Ala   Leu   Ser   Leu   Ala   Leu

      GTC   ACA   AAC   AGT   AAA   AAA   AAC   GGC   TAC   GCT
      Val   Thr   Asn   Ser   Lys   Lys   Asn   Gly   Tyr   Ala

      GTT   GAC   TCT   TCT   GGC   AAA   GCT   CCG   GAA   TGC
      Val   Asp   Ser   Ser   Gly   Lys   Ala   Pro   Glu   Cys

      CTG   CTG   TCT   AAC   TAC   TGC   AAC   AAC   CAG   TGC
      Leu   Leu   Ser   Asn   Tyr   Cys   Asn   Asn   Gln   Cys

      ACT   AAA   GTT   CAT   TAC   GCT   GAC   AAA   GGC   TAC
      Thr   Lys   Val   His   Tyr   Ala   Asp   Lys   Gly   Tyr

      TCG   TGC   CTG   CTG   TCT   TGC   TAC   TGC   TTC   GGC
      Cys   Cys   Leu   Leu   Ser   Cys   Tyr   Cys   Phe   Gly

      CTG   AAC   GAC   GAC   AAA   AAA   GTT   CTG   GAA   ATC
      Leu   Asn   Asp   Asp   Lys   Lys   Val   Leu   Glu   Ile

      TCT   GAC   ACT   CGT   AAA   TCT   TAC   TGC   GAC   ACT
      Ser   Asp   Thr   Arg   Lys   Ser   Tyr   Cys   Asp   Thr

      ACT   ATC   AAC   TAA   TAG - 3'
      Thr   Ile   Asn   *     *
```

# FIG.12
## Restriction Site and Function Map of
## Plasmid pMSV-IT
## ( ~ 12.7 kb )

pMSV-IT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GENE. vol. 73, no. 2, 20 December 1988, AMSTERDAM Nl pages 409 - 418; CARBONELL, L.F. et al.: "Synthesis of a gene coding for an insect-specific scorpion neurotoxin andattempts to express it using baculovirus vectors" * page 413, paragraph C * | 1, 18, 19, 20 | C12N15/12 C12N15/62 C07K13/00 C12N15/85 |
| Y | EP-A-225701 (TAKEDA CHEMICAL INUDSTRIES) * claims * | 1-3, 5, 8, 9, 19, 20 | |
| Y | GB-A-2190382 (HOFFMAN-LA ROCHE) * the whole document * | 1, 2 | |
| Y | Nat. Toxins, Proc. World Congr. Anim., plant microbiol. Toxins,9th meeting 1988, OXFORD, UK pages 94 - 101; BOUGIS, P.E. et al.: "Scorpion venom neurotoxins: cDNA cloning and expression" * the whole document * | 1-9, 12, 14, 19-23 | TECHNICAL FIELDS SEARCHED (Int. Cl.5 ) |
| X | * page 98 * | 18 | C12N C07K |
| D,Y | Insect. Biochem. vol. 13, no. 3, 1983, pages 219 - 236; ZLOTKIN, E.: "Insect selective toxins derived from scorpion venoms : an approach to insect neuropharmacology" * the whole document * | 1-9, 12, 14, 19-23 | |

The present search report has been drawn up for all claims

| Place of search | Date of completion of the search | Examiner |
|---|---|---|
| THE HAGUE | 18 DECEMBER 1990 | CHAMBONNET F.J. |

SEQUENCE LISTING

(1) GENERAL INFORMATION:

    (i) APPLICANT:Eli Lilly and Company

    (ii) TITLE OF INVENTION: Expression of a Functional Insect Specific Toxin Gene In Mammalian Cells

    (iii) NUMBER OF SEQUENCES: 10

    (iv) CORRESPONDENCE ADDRESS:

        (A) ADDRESSEE:Mr. C. Mark Hudson

        (B) STREET:Erl Wood Manor

        (C) CITY:Windlesham

        (D) STATE:Surrey GU20 6PH

        (E) COUNTRY:Grande Bretagne

        (F) ZIP:

(v) COMPUTER READABLE FORM:

    (A) MEDIUM TYPE:Diskette, 3.50 inch, 1.0 Mb storage

    (B) COMPUTER:Macintosh

    (C) OPERATING SYSTEM:Macintosh

    (D) SOFTWARE:Microsoft Word

(vi) CURRENT APPLICATION DATA:

    (A) APPLICATION NUMBER:

    (B) FILING DATE:

    (C) CLASSIFICATION:

(vii) PRIOR APPLICATION DATA:

    (A) APPLICATION NUMBER:

    (B) FILING DATE:

(viii) ATTORNEY/AGENT INFORMATION:

    (A) NAME:Mr. C. Mark Hudson

(B) REGISTRATION NUMBER:307

(C) REFERENCE/DOCKET NUMBER:X-7581

(ix) TELECOMMUNICATION INFORMATION:

(A) TELEPHONE:0276 78441

(B) TELEFAX:0276 78306

(C) TELEX:858177

(2) INFORMATION FOR SEQ ID NO:1 :Sequence found in Claims 4, 7, 14 and 24

(i) SEQUENCE CHARACTERISTICS:

(A) LENGTH: 213 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

    (vii)  IMMEDIATE SOURCE:

        (A)  LIBRARY:

        (B)  CLONE:

    (viii)  POSITION IN GENOME:

        (A)  CHROMOSOME/SEGMENT:

        (B)  MAP POSITION:

        (C)  UNITS:

    (ix)  FEATURE:

           (A)  NAME/KEY:

        (B)  LOCATION:

        (C)  IDENTIFICATION METHOD:

        (D)  OTHER INFORMATION:

     (x)  PUBLICATION INFORMATION:

        (A)  AUTHORS:

        (B)  TITLE:

        (6C)  JOURNAL:

        (D)  VOLUME:

        (E)  ISSUE:

        (F)  PAGES:

        (G)  DATE:

        (H)  DOCUMENT NUMBER:        .

        (I)  FILING DATE:

        (J)  PUBLICATION DATE:

        (K)  RELEVANT RESIDUES:

   (xi)  SEQUENCE DESCRIPTION: SEQ ID NO:1 :

1     AAA AAA AAC GGC TAC GCT GTT GAC TCT TCT     30
       Lys Lys Asn Gly Tyr Ala Val Asp Ser Ser

```
31    GCG AAA GCT CCG GAA TGC CTG CTG TCT AAC    60
      Gly Lys Ala Pro Glu Cys Leu Leu Ser Asn

61    TAC TGC AAC AAC CAG TGC ACT AAA GTT CAT    90
      Tyr Cys Asn Asn Gln Cys Thr Lys Val His

91    TAC GCT GAC AAA GGC TAC TGC TGC CTG CTG    120
      Tyr Ala Asp Lys Gly Tyr Cys Cys Leu Leu

121   TCT TGC TAC TGC TTC GGC CTG AAC GAC GAC    150
      Ser Cys Tyr Cys Phe Gly Leu Asn Asp Asp

151   AAA AAA GTT CTG GAA ATC TCT GAC ACT CGT    180
      Lys Lys Val Leu Glu Ile Ser Asp Thr Arg

181   AAA TCT TAC TGC GAC ACT ACT ATC AAC TAATAG    213
      Lys Ser Tyr Cys Asp Thr Thr Ile Asn
```

(3) INFORMATION FOR SEQ ID NO:2 :Sequence encoding amino acids +1 to +69 of AaIT protein found in specification at pages 12 and 48.

(i) SEQUENCE CHARACTERISTICS:

(A) LENGTH:226 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

        (G) CELL TYPE:

                (H) CELL LINE:

                (I) ORGANELLE:

    (vii) IMMEDIATE SOURCE:

        (A) LIBRARY:

        (B) CLONE:

    (viii) POSITION IN GENOME:

        (A) CHROMOSOME/SEGMENT:

        (B) MAP POSITION:

        (C) UNITS:

    (ix) FEATURE:

                (A) NAME/KEY:

        (B) LOCATION:

        (C) IDENTIFICATION METHOD:

        (D) OTHER INFORMATION:

        (x) PUBLICATION INFORMATION:

        (A) AUTHORS: Zlotkin, E.

        (B) TITLE:

        (C) JOURNAL: Biochemistry

        (D) VOLUME: 13

        (E) ISSUE:

        (F) PAGES:219-236

        (G) DATE: 1983

        (H) DOCUMENT NUMBER:

        (I) FILING DATE:

        (J) PUBLICATION DATE:

EP 0 417 906 A1

(K) RELEVANT RESIDUES:

(xi) SEQUENCE DESCRIPTION: SEQ ID NO:2

```
1        GATCCAAATA ATG AAA AAA AAC GGC TAC GCT        31
                    Met Lys Lys Asn Gly Tyr Ala


32       GTT GAC TCT TCT GGC AAA GCT CCG GAA TGC        61
         Val Asp Ser Ser Gly Lys Ala Pro Glu Cys

62       CTG CTG TCT AAC TAC TGC AAC AAC CAG TGC        91
         Leu Leu Ser Asn Tyr Cys Asn Asn Gln Cys

92       ACT AAA GTT CAT TAC GCT GAC AAA GGC TAC        121
         Thr Lys Val His Tyr Ala Asp Lys Gly Tyr

122      TGC TGC CTG CTG TCC TGC TAC TGC TTC GGC        151
         Cys Cys Leu Leu Ser Cys Tyr Cys Phe Gly

152      CTG AAC GAC GAC AAA AAA GTT CTG GAA ATC        181
         Leu Asn Asp Asp Lys Lys Val Leu Glu Ile

182      TCT GAC ACT CGT AAA TCT TAC TGC GAC ACT        211
         Ser ASp Thr Arg Lys Ser Tyr Cys Asp Thr

212      ACT ATC AAC TAATAG                             226
         Thr Ile Asn
```

(4) INFORMATION FOR SEQ ID NO:3  Sequence  encoding -20 to - 1 of the signal peptide of human interleukin-2 and the first 8 amino acids of AaIT, found on page 13, lines 16-19, and page 49, lines 21-24, of the specification

(i) SEQUENCE CHARACTERISTICS:

(A) LENGTH:  94 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A)  ORGANISM:

(B)  STRAIN:

(C)  INDIVIDUAL ISOLATE:

(D)  DEVELOPMENTAL STAGE:

(E)  HAPLOTYPE:

(F)  TISSUE TYPE:

(G)  CELL TYPE:

(H)  CELL LINE:

(I)  ORGANELLE:

(vii)  IMMEDIATE SOURCE:

(A)  LIBRARY:

(B)  CLONE:

(viii)  POSITION IN GENOME:

(A)  CHROMOSOME/SEGMENT:

(B)  MAP POSITION:

(C)  UNITS:

(ix)  FEATURE:

(A)  NAME/KEY:

(B)  LOCATION:

(C)  IDENTIFICATION METHOD:

(D)  OTHER INFORMATION:

(x)  PUBLICATION INFORMATION:

(A)  AUTHORS:

(B)  TITLE:

(C)  JOURNAL:

(D)  VOLUME:

(E)  ISSUE:

+30                    +35

Ser Cys Tyr Cys Phe Gly Leu Asn Asp Asp
+40                    +45

Lys Lys Val Leu Glu Ile Ser Asp Thr Arg
+50                    +55

Lys Ser Tyr Cys Asp Thr Thr Ile Asn
+60                    +65

(F) PAGES:

(G) DATE:

(H) DOCUMENT NUMBER:

(I) FILING DATE:

(J) PUBLICATION DATE:

(K) RELEVANT RESIDUES:

(xi) SEQUENCE DESCRIPTION: SEQ ID NO:3

```
 1     GA TCTATAAATA ATG TAC AGG ATG CAA CTC     30
                      Met Tyr Arg Met Gln Leu

31     CTG TCT TGC ATT GCA CTA AGT CTT GCA CTT    60
       Leu Ser Lys Ile Ala Leu Ser Leu Ala Leu

61     GTC ACA AAC AGT AAA AAA AAC GGC TAC GCT    90
       Val Thr Asn Ser Lys Lys Asn Gly Tyr Ala

91     GTT G                                      94
       Val
```

(5) INFORMATION FOR SEQ ID NO:4  Sequence encoding amino acids +9 to +69 of AaIT found on pages 13-14 and 50 of the specification.

(i) SEQUENCE CHARACTERISTICS:

(A) LENGTH:  191 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

(vii) IMMEDIATE SOURCE:

(A) LIBRARY:

(B) CLONE:

(viii) POSITION IN GENOME:

(A) CHROMOSOME/SEGMENT:

(B) MAP POSITION:

(C) UNITS:

(ix) FEATURE:

(A) NAME/KEY:

(B) LOCATION:

(C) IDENTIFICATION METHOD:

(D) OTHER INFORMATION:

(x) PUBLICATION INFORMATION:

(A) AUTHORS:

(B) TITLE:

(C) JOURNAL:

(D) VOLUME:

(E) ISSUE:

(F) PAGES:

(G) DATE:

(H) DOCUMENT NUMBER:

(I) FILING DATE:

(J) PUBLICATION DATE:

(K) RELEVANT RESIDUES:

(xi) SEQUENCE DESCRIPTION: SEQ ID NO:4

```
1              AC TCT TCT GGC AAA GCT CCG GAA TGC    26
               Ser Ser Gly Lys Ala Pro Glu Cys

27   CTG CTG TCT AAC TAC TGC AAC AAC CAG TGC         56
     Leu Leu Ser Asn Tyr Cys Asn Asn Gln Cys

57   ACT AAA GTT CAT TAC GCT GAC AAA GGC TAC         86
     Thr Lys Val His Tyr Ala Asp Lys Gly Tyr

87   TGC TGC CTG CTG TCT TGC TAC TGC TTC GGC         116
     Cys Cys Leu Leu Ser Cys Tyr Cys Phe Gly

117  CTG AAC GAC GAC AAA AAA GTT CTG GAA ATC         146
     Leu Asn Asp Asp Lys Lys Val Leu Glu Ile

147  TCT GAC ACT CGT AAA TCT TAC TGC GAC ACT         176
     Ser ASp Thr Arg Lys Ser Tyr Cys Asp Thr

177  ACT ATC AAC TAATAG                             191
     Thr Ile Asn
```

(6) INFORMATION FOR SEQ ID NO:5   DNA linker sequence found on page 40 of the specification.

(i) SEQUENCE CHARACTERISTICS:

(A) LENGTH:  21 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

    (vi) ORIGINAL SOURCE:

    (A) ORGANISM:

    (B) STRAIN:

    (C) INDIVIDUAL ISOLATE:

    (D) DEVELOPMENTAL STAGE:

    (E) HAPLOTYPE:

    (F) TISSUE TYPE:

    (G) CELL TYPE:

    (H) CELL LINE:

    (I) ORGANELLE:

(vii) IMMEDIATE SOURCE:

    (A) LIBRARY:

    (B) CLONE:

(viii) POSITION IN GENOME:

    (A) CHROMOSOME/SEGMENT:

    (B) MAP POSITION:

    (C) UNITS:

(ix) FEATURE:

    (A) NAME/KEY:

    (B) LOCATION:

    (C) IDENTIFICATION METHOD:

    (D) OTHER INFORMATION:

    (x) PUBLICATION INFORMATION:

    (A) AUTHORS:

    (B) TITLE:

    (C) JOURNAL:

(D) VOLUME:

(E) ISSUE:

(F) PAGES:

(G) DATE:

(H) DOCUMENT NUMBER:

(I) FILING DATE:

(J) PUBLICATION DATE:

(K) RELEVANT RESIDUES:

(xi) SEQUENCE DESCRIPTION: SEQ ID NO:5

1    GATCTATTAACTCAATCTAGAC    21

(7) INFORMATION FOR SEQ ID NO:6   BamHI linker sequence shown on page 42 of the specification at line 20.

(i) SEQUENCE CHARACTERISTICS:

(A) LENGTH:   10 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E)  HAPLOTYPE:

(F)  TISSUE TYPE:

(G)  CELL TYPE:

(H)  CELL LINE:

(I)  ORGANELLE:

(vii) IMMEDIATE SOURCE:

(A)  LIBRARY:

(B)  CLONE:

(viii) POSITION IN GENOME:

(A)  CHROMOSOME/SEGMENT:

(B)  MAP POSITION:

(C)  UNITS:

(ix) FEATURE:

(A)  NAME/KEY:

(B)  LOCATION:

(C)  IDENTIFICATION METHOD:

(D)  OTHER INFORMATION:

(x) PUBLICATION INFORMATION:

(A)  AUTHORS:

(B)  TITLE:

(C)  JOURNAL:

(D)  VOLUME:

(E)  ISSUE:

(F)  PAGES:

(G)  DATE:

(H)  DOCUMENT NUMBER:

(I)  FILING DATE:

(J) PUBLICATION DATE:

(K) RELEVANT RESIDUES:

(xi) SEQUENCE DESCRIPTION: SEQ ID NO:6

1          CGGGATCCCG          10

(8) INFORMATION FOR SEQ ID NO:7   EcoRI linker sequence found on page 43, line 18 of the specification.

(i) SEQUENCE CHARACTERISTICS:

(A) LENGTH:   12 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

(vii) IMMEDIATE SOURCE:

(A) LIBRARY:

             (B) CLONE:

     (viii) POSITION IN GENOME:

             (A) CHROMOSOME/SEGMENT:

             (B) MAP POSITION:

             (C) UNITS:

         (ix) FEATURE:

                 (A) NAME/KEY:

             (B) LOCATION:

             (C) IDENTIFICATION METHOD:

             (D) OTHER INFORMATION:

         (x) PUBLICATION INFORMATION:

             (A) AUTHORS:

             (B) TITLE:

             (C) JOURNAL:

             (D) VOLUME:

             (E) ISSUE:

             (F) PAGES:

             (G) DATE:

             (H) DOCUMENT NUMBER:

             (I) FILING DATE:

             (J) PUBLICATION DATE:

             (K) RELEVANT RESIDUES:

     (xi) SEQUENCE DESCRIPTION: SEQ ID NO:7

     1      GAGGAATTCCTC          12

     (9) INFORMATION FOR SEQ ID NO:8    BamHI linker sequence
     found on page 46, line 22 of the specification.

         (i) SEQUENCE CHARACTERISTICS:

```
        (A) LENGTH:  13 Nucleotides

        (B) TYPE:

            (C) STRANDEDNESSS:

            (D) TOPOLOGY:

    (ii) MOLECULE TYPE: DNA

    (iii) HYPOTHETICAL:

    (iv) ANTI-SENSE:

    (v) FRAGMENT TYPE:

        (vi) ORIGINAL SOURCE:

        (A) ORGANISM:

        (B) STRAIN:

        (C) INDIVIDUAL ISOLATE:

        (D) DEVELOPMENTAL STAGE:

            (E) HAPLOTYPE:

        (F) TISSUE TYPE:

        (G) CELL TYPE:

            (H) CELL LINE:

            (I) ORGANELLE:

    (vii) IMMEDIATE SOURCE:

        (A) LIBRARY:

        (B) CLONE:

    (viii) POSITION IN GENOME:

        (A) CHROMOSOME/SEGMENT:

        (B) MAP POSITION:

        (C) UNITS:

    (ix) FEATURE:

            (A) NAME/KEY:
```

(B) LOCATION:

(C) IDENTIFICATION METHOD:

(D) OTHER INFORMATION:

(x) PUBLICATION INFORMATION:

(A) AUTHORS:

(B) TITLE:

(C) JOURNAL:

(D) VOLUME:

(E) ISSUE:

(F) PAGES:

(G) DATE:

(H) DOCUMENT NUMBER:

(I) FILING DATE:

(J) PUBLICATION DATE:

(K) RELEVANT RESIDUES:

(xi) SEQUENCE DESCRIPTION: SEQ ID NO:8

1     CTGTGCCTTCTAG       13

(10) INFORMATION FOR SEQ ID NO:9  Sequence ecoding the synthetic SP-IT gene found on page 51 of the specification.

(i) SEQUENCE CHARACTERISTICS:

(A) LENGTH:  285 Nucleotides

(B) TYPE:

(C) STRANDEDNESSS:

(D) TOPOLOGY:

(ii) MOLECULE TYPE: DNA

(iii) HYPOTHETICAL:

(iv) ANTI-SENSE:

(v)  FRAGMENT TYPE:

  (vi)  ORIGINAL SOURCE:

    (A)  ORGANISM:

    (B)  STRAIN:

    (C)  INDIVIDUAL ISOLATE:

    (D)  DEVELOPMENTAL STAGE:

      (E)  HAPLOTYPE:

    (F)  TISSUE TYPE:

    (G)  CELL TYPE:

      (H)  CELL LINE:

      (I)  ORGANELLE:

  (vii)  IMMEDIATE SOURCE:

    (A)  LIBRARY:

    (B)  CLONE:

  (viii)  POSITION IN GENOME:

    (A)  CHROMOSOME/SEGMENT:

    (B)  MAP POSITION:

    (C)  UNITS:

  (ix)  FEATURE:

      (A)  NAME/KEY:

    (B)  LOCATION:

    (C)  IDENTIFICATION METHOD:

    (D)  OTHER INFORMATION:

  (x)  PUBLICATION INFORMATION:

    (A)  AUTHORS:

    (B)  TITLE:

    (C)  JOURNAL:

(D)  VOLUME:

(E)  ISSUE:

(F)  PAGES:

(G)  DATE:

(H)  DOCUMENT NUMBER:

(I)  FILING DATE:

(J)  PUBLICATION DATE:

(K)  RELEVANT RESIDUES:

(xi) SEQUENCE DESCRIPTION: SEQ ID NO:9

```
  1  GA TCTATAAATA ATG TAC AGG ATG CAA CTC        30
                    Met Tyr Arg Met Gln Leu

 31        CTG TCT TGC ATT GCA CTA AGT CTT GCA     57
           Leu Ser Lys Ile Ala Leu Ser Leu Ala

 58        CTT GTC ACA AAC AGT AAA AAA AAC GGC     84
           Leu Val Thr Asn Ser Lys Lys Asn Gly

 85        TAC GCT GTT GAC TCT TCT GGC AAA GCT     111
           Tyr Ala Val Asp Ser Ser Gly Lys Ala

112        CCG GAA TGC CTG CTG TCT AAC TAC TGC     138
           Pro Glu Cys Leu Leu Ser Asn Tyr Cys

139        AAC AAC CAG TGC ACT AAA GTT CAT TAC     165
           Asn Asn Gln Cys Thr Lys Val His Tyr

166        GCT GAC AAA GGC TAC TCG TGC CTG CTG     192
           Ala Asp Lys Gly Tyr Cys Cys Leu Leu

193        TCT TGC TAC TGC TTC GGC CTG AAC GAC     219
           Ser Cys Tyr Cys Phe Gly Leu Asn Asp

220        GAC AAA AAA GTT CTG GAA ATC TCT GAC     246
           Asp Lys Lys Val Leu Glu Ile Ser Asp

247        ACT CGT AAA TCT TAC TGC GAC ACT ACT     273
           Thr Arg Lys Ser Tyr Cys Asp Thr Thr

274        ATC AAC TAATAG                         285
           Ile Asn
```

(11) INFORMATION FOR SEQ ID NO:10  Amino acid sequence of the SP-IT fusion protein found on page 51 of the specification.

(i) SEQUENCE CHARACTERISTICS:

(A) LENGTH:  89 amino acids

(B) TYPE:

(C) STRANDEDNESSS:

(D) TOPOLOGY:

(ii) MOLECULE TYPE: protein

(iii) HYPOTHETICAL:

(iv) ANTI-SENSE:

(v) FRAGMENT TYPE:

(vi) ORIGINAL SOURCE:

(A) ORGANISM:

(B) STRAIN:

(C) INDIVIDUAL ISOLATE:

(D) DEVELOPMENTAL STAGE:

(E) HAPLOTYPE:

(F) TISSUE TYPE:

(G) CELL TYPE:

(H) CELL LINE:

(I) ORGANELLE:

(vii) IMMEDIATE SOURCE:

(A) LIBRARY:

(B) CLONE:

(viii) POSITION IN GENOME:

(A) CHROMOSOME/SEGMENT:

(B) MAP POSITION:

```
        (C) UNITS:

    (ix) FEATURE:

            (A) NAME/KEY:

        (B) LOCATION:

        (C) IDENTIFICATION METHOD:

        (D) OTHER INFORMATION:

     (x) PUBLICATION INFORMATION:

        (A) AUTHORS:

        (B) TITLE:

        (C) JOURNAL:

        (D) VOLUME:

        (E) ISSUE:

        (F) PAGES:

        (G) DATE:

        (H) DOCUMENT NUMBER:

        (I) FILING DATE:

        (J) PUBLICATION DATE:

        (K) RELEVANT RESIDUES:

    (xi) SEQUENCE DESCRIPTION: SEQ ID NO:11

    Met Tyr Arg Met Gln Leu Leu Ser Lys Ile
    -20               -15

    Ala Leu Ser Leu Ala Leu Val Thr Asn Ser
    -10                    -5

    Lys Lys Asn Gly Tyr Ala Val Asp Ser Ser
     +1                    +5

    Gly Lys Ala Pro Glu Cys Leu Leu Ser Asn
    +10                    +15

    Tyr Cys Asn Asn Gln Cys Thr Lys Val His
    +20  .                 +25

    Tyr Ala Asp Lys Gly Tyr Cys Cys Leu Leu
```